# EUROPEAN PATENT APPLICATION

(11) **EP 4 785 943 A1**
(43) Date of publication of application: **05.08.2026**
(21) Application number: 24870987.5
(22) Date of filing: 27.09.2024
(51) Int. Cl.: A61K 31/498, A61K 9/20, A61K 47/32, A61P 35/00, A61P 37/00, A61P 25/28

(54) **SOLID DISPERSION, PHARMACEUTICAL COMPOSITION, AND PREPARATION METHOD AND USE THEREFOR**

(30) Priority: 28.09.2023 WO PCT/CN2023/122859
(71) Applicant: 4B Technologies (Beijing) Co., Limited, Beijing 100176 (CN)
(72) Inventor: LIU, Changhai, Suzhou, Jiangsu 215123 (CN); LU, Gang, Suzhou, Jiangsu 215123 (CN)
(74) Representative: Haseltine Lake Kempner LLP
(86) International application number: PCT/CN2024/121819
(87) International publication number: WO 2025/067450

(57) **Abstract**

A solid dispersion, a pharmaceutical composition, and preparation method and use therefor. The solid dispersion comprises the following components: a compound 1 and a polymer carrier, wherein the polymer carrier is selected from one or more of HPMCAS, Soluplus and PVP-VA. The solid dispersion is more beneficial to dispersing the compound 1 in the polymer carrier in an amorphous or near-amorphous or non-crystalline state, and is beneficial to improving the solubility of the compound 1.

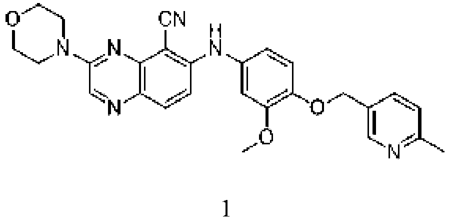

## Description

The present application claims priority to PCT international patent application No. PCT/CN2023/122859, filed on September 28, 2023. The contents of the above PCT international patent application are incorporated herein by reference in their entireties.

### TECHNICAL FIELD

The present disclosure relates to a solid dispersion, a pharmaceutical composition, a preparation method therefor, and the use thereof.

### BACKGROUND

Compound 1 (see US10689362B2, Compound 54) is a type III receptor tyrosine kinase inhibitor capable of inhibiting kinases and is used for treating diseases or disorders mediated by these kinases. Compound 1 inhibits CSF-1R kinase (IC₅₀ < 100 nM). Studies have found that Compound 1 is poorly soluble in aqueous media and exhibits pH-dependent solubility, necessitating the development of a formulation with high solubility and high bioavailability.

In drug development programs, many new drug molecules exhibit poor oral bioavailability. A drug molecule may have low oral bioavailability due to (1) poor dissolution rate, (2) poor solubility, or (3) limited permeability. Depending on the etiology, different strategies have been adopted to improve the absorption of oral drugs. The bioavailability of drug substances limited by dissolution rate can be improved through various solubilization techniques, for example, by preparing solid dispersions with polymeric materials, or using large amounts of surfactants, cyclodextrins, micelles, polymeric micelles, liposomes, acidic pH adjusters, or dendrimers.

Solid dispersions are solid substances proposed in 1961 in which poorly soluble drugs are highly dispersed in suitable solid carrier materials. The solubility and dispersion state of drugs are among the main factors affecting their *in vivo* bioavailability. Solid dispersion technology enables drugs to form a highly dispersed state in polymeric carriers, which can effectively increase the solubility and dissolution rate of poorly soluble drugs, thereby enhancing their bioavailability.

There are various preparation methods of solid dispersions with numerous optional excipients. Although many publications on solid dispersions have been reported, the suitable formulations and preparation processes vary greatly depending on the specific properties of the drug, requiring research and experimentation based on the characteristics of the drug.

Optimizing formulations can also improve the solubility and dissolution rate of poorly soluble drugs, thereby enhancing their bioavailability. Due to the relatively complex solubilization mechanisms of optimized formulations with more numerous optional excipients, it is necessary to experiment with different mechanisms of action based on the characteristics of the drug and select various excipients for experimentation and research.

To date, no solubilization techniques for Compound 1 have been reported. 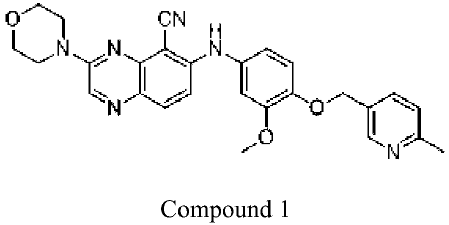
Chemical name: 6-((3-methoxy-4-((6-methylpyridin-3-yl)methoxy)phenyl)amino)-3-morpholinoquinoxaline-5-carbonitrile;
Molecular formula: C₂₇H₂₆N₆O₃.

### SUMMARY

The technical problem to be solved by the present disclosure is to overcome one or both of the disadvantages of low solubility and low *in vivo* exposure of Compound 1, and further to overcome one or both of the disadvantages of high production cost and poor patient compliance when Compound 1 is used as a medicament. Accordingly, the present disclosure provides a solid dispersion, a pharmaceutical composition, a preparation method therefor, and the use thereof. The pharmaceutical composition of the present disclosure provides one or both of the advantages of good solubility and good *in vivo* exposure, and further provides one or both of the advantages of low production cost and good patient compliance.

The present disclosure mainly solves the above technical problems through the following technical solutions.

The present disclosure provides a solid dispersion comprising the following components: Compound 1 and a polymeric carrier; 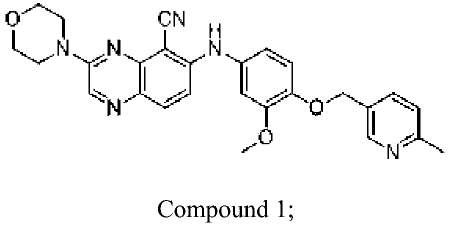 wherein the polymeric carrier is selected from one or more of HPMCAS, Soluplus, and PVP-VA.

In some embodiments, in the solid dispersion, the polymeric carrier is HPMCAS.

In some embodiments, in the solid dispersion, the polymeric carrier is Soluplus.

In some embodiments, in the solid dispersion, the polymeric carrier is PVP-VA.

In some embodiments, in the solid dispersion, the HPMCAS is preferably selected from one or both of HPMCAS-HF and HPMCAS-HG, more preferably HPMCAS-HG. The PVP-VA is preferably PVP-VA64. The PVP-VA64 may be Plasdone^{TM} S-630 or Plasdone^{TM} S-630 ULTRA.

In some embodiments, in the solid dispersion, the weight ratio of Compound 1 to the polymeric carrier is approximately 1:1 to approximately 1:10.

In some embodiments, in the solid dispersion, the weight ratio of Compound 1 to the polymeric carrier is preferably approximately 1:2 to approximately 1:5, such as 3:7, 1:3, or 1:4, more preferably approximately 1:3 to approximately 1:4, and even more preferably approximately 1:3.

In some embodiments, in the solid dispersion, the weight ratio of Compound 1 to the polymeric carrier is approximately 3:7.

In some embodiments, in the solid dispersion, the polymeric carrier is present in an amount of at least approximately 50%, at least approximately 60%, at least approximately 67%, at least approximately 70%, at least approximately 75%, at least approximately 80%, or at least approximately 90% by weight of the solid dispersion.

In some embodiments, in the solid dispersion, Compound 1 is present in an amount of approximately 10% to approximately 50%, approximately 20% to approximately 40%, or approximately 25% to approximately 33% by weight of the solid dispersion.

In some embodiments, in addition to the polymeric carrier, the solid dispersion of the present disclosure may further comprise other excipients, such as surfactants, glidants, and plasticizers.

In some embodiments, the solid dispersion of the present disclosure may further comprise a surfactant.

In some embodiments, in the solid dispersion, Compound 1 is dispersed in the polymeric carrier in an amorphous (or near-amorphous) or substantially non-crystalline state.

In some embodiments, Compound 1 in the solid dispersion is substantially amorphous.

In some embodiments, the solid dispersion is obtained by spray drying, hot-melt extrusion, or solvent evaporation.

In some embodiments, the solid dispersion consists of Compound 1 and the polymeric carrier.

In some embodiments, the solid dispersion consists of components in mass percentages according to any one of the following schemes:
scheme 1.1: 25% Compound 1 and 75% Soluplus;
scheme 1.2: 25% Compound 1 and 75% Plasdone^{TM} S-630 ULTRA;
scheme 1.3: 20% Compound 1 and 80% Soluplus;
scheme 1.4: 25% Compound 1 and 75% HPMCAS-HG;
scheme 1.5: 30% Compound 1 and 70% HPMCAS-HG.

The present disclosure further provides a preparation method of the solid dispersion according to any one of the foregoing embodiments of the present disclosure, comprising the step of mixing the components.

In some embodiments, the preparation method of the solid dispersion preferably comprises the steps of: dissolving Compound 1 and the polymeric carrier in a solvent, mixing uniformly, and evaporating the solvent to obtain the solid dispersion.

In some embodiments, the solvent is evaporated by spray drying in the preparation method.

In some embodiments, the solvent used in the preparation method is an organic solvent.

In some embodiments, the organic solvent is selected from one or both of halogenated alkane solvents and alcohol solvents, preferably a mixture of dichloromethane and ethanol.

The present disclosure further provides a solid dispersion prepared by the above preparation method.

The present disclosure further provides a pharmaceutical composition comprising the solid dispersion according to any one of the foregoing embodiments of the present disclosure, and one or more pharmaceutically acceptable carriers.

In some embodiments, the excipient in the solid dispersion and the pharmaceutically acceptable carrier in the pharmaceutical composition are different from each other.

In some embodiments, the pharmaceutical composition is a tablet, a capsule, a granule, a powder, a dry suspension, or an enteric-coated preparation.

In some embodiments, the pharmaceutically acceptable carrier in the pharmaceutical composition is selected from one or more of a solubilizer, an acidic pH adjuster, a disintegrant, a glidant, a filler, a lubricant, and a surfactant.

In some embodiments, the pharmaceutical composition comprises the following components in mass percentages:
20% to 70% of the solid dispersion according to any one of the foregoing embodiments of the present disclosure,
0% to 30% of a solubilizer,
0% to 20% of an acidic pH adjuster,
3% to 30% of a disintegrant,
0% to 10% of a glidant,
0% to 50% of a filler,
0% to 1% of a lubricant, and
0% to 20% of a surfactant.

In the present disclosure, one or more of the polymeric carrier, the solubilizer, the acidic pH adjuster, and the surfactant function to improve the solubility of Compound 1.

In the present disclosure, the solid dispersion functions to improve the solubility of Compound 1.

In some embodiments, when the mass percentage of the solubilizer is 0%, the mass percentage of the acidic pH adjuster is not 0%.

In some embodiments, in the pharmaceutical composition, the mass percentage of the solid dispersion is 40%, 50%, 64.5%, or 66.4%.

In some embodiments, in the pharmaceutical composition, the mass percentage of the solid dispersion is 30% to 60%, preferably 40% to 50%.

In some embodiments, in the pharmaceutical composition, the mass percentage of the solubilizer is, for example, 0%, 5.8%, 6%, 7.5%, 12%, 14%, 17.5%, or 24%.

In some embodiments, in the pharmaceutical composition, the mass percentage of the solubilizer is preferably 0% to 24%.

In some embodiments, in the pharmaceutical composition, the mass percentage of the solubilizer is preferably 4% to 14%, such as 6%, 7.5%, or 12%.

In some embodiments, in the pharmaceutical composition, the mass percentage of the acidic pH adjuster is, for example, 0%, 5%, 6.25%, 10%, 15%, 20%, or 39.5%.

In some embodiments, in the pharmaceutical composition, the mass percentage of the acidic pH adjuster is preferably 0% to 15%, such as 0%, 5%, 6.25%, or 10%.

In some embodiments, in the pharmaceutical composition, the mass percentage of the acidic pH adjuster is more preferably 3% to 12%, such as 5%, 6.25%, or 10%.

In some embodiments, in the pharmaceutical composition, the mass percentage of the disintegrant is, for example, 3%, 8%, 10%, 14%, 26.4%, or 27.2%.

In some embodiments, in the pharmaceutical composition, the mass percentage of the disintegrant is preferably 3% to 20%, more preferably 3% to 15%, such as 3% or 10%.

In some embodiments, in the pharmaceutical composition, the mass percentage of the glidant is, for example, 0% or 4%.

In some embodiments, in the pharmaceutical composition, the mass percentage of the glidant is preferably 1% to 10%, such as 4%.

In some embodiments, in the pharmaceutical composition, the mass percentage of the filler is, for example, 0%, 13.5%, 16.5%, 17.5%, 18.50%, 19.25%, 19.5%, 21.5%, 22.5%, 25.5%, 26.5%, 28.5%, 29.25%, 29.5%, 30.5%, 34.5%, 35.5%, 36.5%, 39.5%, or 40.5%.

In some embodiments, in the pharmaceutical composition, the mass percentage of the filler is preferably 10% to 45%, more preferably 20% to 40%, such as 28.5%, 29.25%, or 34.5%.

In the present disclosure, in the pharmaceutical composition, the mass percentage of the filler may be increased or decreased accordingly depending on adjustments of other components.

In some embodiments, in the pharmaceutical composition, the mass percentage of the lubricant is, for example, 0%, 0.4%, or 0.5%, preferably 0% or 0.5%.

In some embodiments, in the pharmaceutical composition, the mass percentage of the surfactant is 0% to 12%.

In some embodiments, in the pharmaceutical composition, the mass percentage of the surfactant is 0% to 10%, such as 0%, 1%, 5%, or 10%.

In some embodiments, in the pharmaceutical composition, the mass percentage of the surfactant is preferably 0% to 5%, such as 0%, 1%, or 5%.

In some embodiments, in the pharmaceutical composition, the mass ratio of the polymeric carrier to Compound 1 is 2:1 to 4:1, such as 2.3:1, 3:1, or 4:1.

In some embodiments, in the pharmaceutical composition, the mass ratio of the polymeric carrier to Compound 1 is preferably 2:1 to 2.5:1, such as 2.3:1.

In some embodiments, in the pharmaceutical composition, the mass ratio of the acidic pH adjuster to Compound 1 is preferably 1:1 to 1:3, such as 1:1.2 or 1:2.4.

In some embodiments, in the pharmaceutical composition, the solubilizer is selected from one or more of PEG, PVP, HPMCAS, HPMC, Eudragit, and Soluplus.

In some embodiments, in the pharmaceutical composition, the solubilizer is selected from one or more of PVP, HPMC, and Soluplus, preferably PVP or Soluplus.

In some embodiments, in the pharmaceutical composition, in the solubilizer, the PEG is selected from one or both of PEG4000 and PEG6000.

In some embodiments, in the pharmaceutical composition, in the solubilizer, the PVP is selected from one or more of PVP K17, PVP K30, PVP K90, and PVP-VA64, preferably PVP-VA64.

In some embodiments, in the pharmaceutical composition, the PVP-VA64 may be Plasdone^{TM} S-630 or Plasdone^{TM} S-630 ULTRA.

In some embodiments, in the pharmaceutical composition, in the solubilizer, the HPMC is preferably HPMC E3LV.

In some embodiments, in the pharmaceutical composition, in the solubilizer, the HPMCAS is selected from one or more of HPMCAS-LF, HPMCAS-MF, HPMCAS-HF, HPMCAS-LG, HPMCAS-MG, and HPMCAS-HG, preferably HPMCAS-HG.

In some embodiments, in the pharmaceutical composition, in the solubilizer, the Eudragit is selected from one or both of Eudragit L100-55 and Eudragit S100.

In some embodiments, in the pharmaceutical composition, the solubilizer is preferably selected from one or more of PVP-VA64, Soluplus, HPMC E3LV, and PVP K30, more preferably PVP-VA64 or Soluplus.

In some embodiments, in the pharmaceutical composition, the acidic pH adjuster is selected from one or both of an organic acid and an inorganic acid. The organic acid is, for example, selected from one or more of citric acid, fumaric acid, malic acid, maleic acid, tartaric acid, succinic acid, oxalic acid, aspartic acid, mandelic acid, glutaric acid, and glutamic acid, preferably selected from one or more of fumaric acid, citric acid, tartaric acid, and malic acid, more preferably tartaric acid. The inorganic acid is preferably selected from one or more of hydrochloric acid, phosphoric acid, nitric acid, and sulfuric acid.

In some embodiments, in the pharmaceutical composition, the acidic pH adjuster is preferably selected from one or more of citric acid, fumaric acid, malic acid, and tartaric acid.

In some embodiments, in the pharmaceutical composition, the acidic pH adjuster is preferably tartaric acid.

In some embodiments, in the pharmaceutical composition, when the acidic pH adjuster is tartaric acid, the mass percentage of the acidic pH adjuster is preferably 3% to 7%, more preferably 4% to 6%, such as 5%.

In some embodiments, in the pharmaceutical composition, when the acidic pH adjuster is malic acid, the mass percentage of the acidic pH adjuster is preferably 3% to 7%, more preferably 4% to 6%, such as 5%.

In some embodiments, in the pharmaceutical composition, when the acidic pH adjuster is fumaric acid, the mass percentage of the acidic pH adjuster is preferably 8% to 12%, more preferably 9% to 11%, such as 10%.

In some embodiments, in the pharmaceutical composition, when the acidic pH adjuster is citric acid, the mass percentage of the acidic pH adjuster is preferably 8% to 12%, more preferably 9% to 11%, such as 10%.

In some embodiments, in the pharmaceutical composition, the disintegrant is selected from one or more of CCMC-Na, CMS-Na, and CMC-Na, preferably one or both of CCMC-Na and CMS-Na.

In some embodiments, in the pharmaceutical composition, the disintegrant is preferably CMC-Na, or a mixture of CCMC-Na and CMS-Na.

In some embodiments, in the pharmaceutical composition, when the disintegrant is a mixture of CCMC-Na and CMS-Na, the mass ratio of CCMC-Na to CMS-Na is 1:1 to 2:1, preferably 1.5:1.

In some embodiments, in the pharmaceutical composition, the glidant is, for example, colloidal silicon dioxide.

In some embodiments, in the pharmaceutical composition, the filler is selected from one or more of mannitol, lactose, compressible starch, and microcrystalline cellulose, preferably mannitol.

In some embodiments, in the pharmaceutical composition, the lubricant is, for example, magnesium stearate.

In some embodiments, in the pharmaceutical composition, the surfactant is, for example, selected from one or more of sodium dodecyl sulfate, vitamin E polyethylene glycol succinate, poloxamer 188, and poloxamer 407.

In some embodiments, the pharmaceutical composition consists of the solid dispersion, the solubilizer, the acidic pH adjuster, the disintegrant, the glidant, the filler, the lubricant, and the surfactant.

In some embodiments, the pharmaceutical composition consists of components in mass percentages according to any one of the following schemes:
scheme 2.1: 19.35% Compound 1, 45.15% HPMCAS-HG, 5.8% PVP K90, 26.4% CMS-Na, 0.4% magnesium stearate, and 2.9% film coating premix (gastric-soluble);
scheme 2.2: 15% Compound 1, 35% HPMCAS-HG, 17.5% PVP-VA64, 4% colloidal silicon dioxide, 19.25% mannitol, 3% CMC-Na, and 6.25% tartaric acid;
scheme 2.3: 15% Compound 1, 35% HPMCAS-HG, 7.5% Soluplus, 4% colloidal silicon dioxide, 29.25% mannitol, 3% CMC-Na, and 6.25% tartaric acid;
scheme 2.4: 15% Compound 1, 35% HPMCAS-HG, 17.5% Soluplus, 4% colloidal silicon dioxide, 19.25% mannitol, 3% CMC-Na, and 6.25% tartaric acid;
scheme 2.5: 12% Compound 1, 28% HPMCAS-HG, 6% PVP-VA64, 4% colloidal silicon dioxide, 34.5% mannitol, 6% CCMC-Na, 4% CMS-Na, 5% tartaric acid, and 0.5% magnesium stearate;
scheme 2.6: 12% Compound 1, 28% HPMCAS-HG, 6% PVP-VA64, 4% colloidal silicon dioxide, 39.5% mannitol, 6% CCMC-Na, 4% CMS-Na, and 0.5% magnesium stearate;
scheme 2.7: 12% Compound 1, 28% HPMCAS-HG, 6% PVP-VA64, 4% colloidal silicon dioxide, 29.5% mannitol, 6% CCMC-Na, 4% CMS-Na, 10% tartaric acid, and 0.5% magnesium stearate;
scheme 2.8: 12% Compound 1, 28% HPMCAS-HG, 6% PVP-VA64, 4% colloidal silicon dioxide, 19.5% mannitol, 6% CCMC-Na, 4% CMS-Na, 20% tartaric acid, and 0.5% magnesium stearate;
scheme 2.9: 12% Compound 1, 28% HPMCAS-HG, 6% PVP-VA64, 4% colloidal silicon dioxide, 6% CCMC-Na, 4% CMS-Na, 39.5% tartaric acid, and 0.5% magnesium stearate;
scheme 2.10: 12% Compound 1, 28% HPMCAS-HG, 6% PVP-VA64, 4% colloidal silicon dioxide, 34.5% mannitol, 6% CCMC-Na, 4% CMS-Na, 5% fumaric acid, and 0.5% magnesium stearate;
scheme 2.11: 12% Compound 1, 28% HPMCAS-HG, 6% PVP-VA64, 4% colloidal silicon dioxide, 34.5% mannitol, 6% CCMC-Na, 4% CMS-Na, 5% citric acid, and 0.5% magnesium stearate;
scheme 2.12: 12% Compound 1, 28% HPMCAS-HG, 6% PVP-VA64, 4% colloidal silicon dioxide, 34.5% mannitol, 6% CCMC-Na, 4% CMS-Na, 5% malic acid, and 0.5% magnesium stearate;
scheme 2.13: 12% Compound 1, 28% HPMCAS-HG, 6% Soluplus, 4% colloidal silicon dioxide, 34.5% mannitol, 6% CCMC-Na, 4% CMS-Na, 5% tartaric acid, and 0.5% magnesium stearate;
scheme 2.14: 12% Compound 1, 28% HPMCAS-HG, 6% HPMC E3LV, 4% colloidal silicon dioxide, 34.5% mannitol, 6% CCMC-Na, 4% CMS-Na, 5% tartaric acid, and 0.5% magnesium stearate;
scheme 2.15: 12% Compound 1, 28% HPMCAS-HG, 6% PVP K30, 4% colloidal silicon dioxide, 34.5% mannitol, 6% CCMC-Na, 4% CMS-Na, 5% tartaric acid, and 0.5% magnesium stearate;
scheme 2.16: 12% Compound 1, 28% HPMCAS-HG, 4% colloidal silicon dioxide, 40.5% mannitol, 6% CCMC-Na, 4% CMS-Na, 5% citric acid, and 0.5% magnesium stearate;
scheme 2.17: 12% Compound 1, 28% HPMCAS-HG, 12% PVP-VA64, 4% colloidal silicon dioxide, 28.5% mannitol, 6% CCMC-Na, 4% CMS-Na, 5% citric acid, and 0.5% magnesium stearate;
scheme 2.18: 12% Compound 1, 28% HPMCAS-HG, 24% PVP-VA64, 4% colloidal silicon dioxide, 16.5% mannitol, 6% CCMC-Na, 4% CMS-Na, 5% citric acid, and 0.5% magnesium stearate;
scheme 2.19: 12% Compound 1, 28% HPMCAS-HG, 14% Soluplus, 4% colloidal silicon dioxide, 26.5% mannitol, 6% CCMC-Na, 4% CMS-Na, 5% tartaric acid, and 0.5% magnesium stearate;
scheme 2.20: 12% Compound 1, 28% HPMCAS-HG, 4% colloidal silicon dioxide, 35.5% mannitol, 6% CCMC-Na, 4% CMS-Na, 5% tartaric acid, 5% sodium lauryl sulfate, and 0.5% magnesium stearate;
scheme 2.21: 12% Compound 1, 28% HPMCAS-HG, 14% Soluplus, 25.5% mannitol, 6% CCMC-Na, 4% CMS-Na, 5% tartaric acid, 5% sodium lauryl sulfate, and 0.5% magnesium stearate;
scheme 2.22: 12% Compound 1, 28% HPMCAS-HG, 14% Soluplus, 4% colloidal silicon dioxide, 26.5% mannitol, 6% CCMC-Na, 4% CMS-Na, 5% sodium lauryl sulfate, and 0.5% magnesium stearate;
scheme 2.23: 19.92% Compound 1, 46.48% HPMCAS-HG, 6% PVP K30, 27.2% CMS-Na, and 0.4% magnesium stearate;
scheme 2.24: 12% Compound 1, 28% HPMCAS-HG, 14% Soluplus, 4% colloidal silicon dioxide, 26.5% mannitol, 10% CMS-Na, 5% tartaric acid, and 0.5% magnesium stearate;
scheme 2.25: 12% Compound 1, 28% HPMCAS-HG, 14% Soluplus, 4% colloidal silicon dioxide, 21.5% mannitol, 6% CCMC-Na, 4% CMS-Na, 5% tartaric acid, 5% sodium lauryl sulfate, and 0.5% magnesium stearate;
scheme 2.26: 12% Compound 1, 28% HPMCAS-HG, 14% PVP-VA64, 4% colloidal silicon dioxide, 21.5% mannitol, 6% CCMC-Na, 4% CMS-Na, 5% tartaric acid, 5% sodium lauryl sulfate, and 0.5% magnesium stearate;
scheme 2.27: 12% Compound 1, 28% HPMCAS-HG, 6% PVP-VA64, 4% colloidal silicon dioxide, 29.5% mannitol, 6% CCMC-Na, 4% CMS-Na, 5% tartaric acid, 5% sodium lauryl sulfate, and 0.5% magnesium stearate;
scheme 2.28: 12% Compound 1, 28% HPMCAS-HG, 6% PVP-VA64, 4% colloidal silicon dioxide, 36.5% mannitol, 6% CCMC-Na, 2% CMS-Na, 5% tartaric acid, and 0.5% magnesium stearate;
scheme 2.29: 12% Compound 1, 28% HPMCAS-HG, 6% PVP-VA64, 4% colloidal silicon dioxide, 30.5% mannitol, 6% CCMC-Na, 8% CMS-Na, 5% tartaric acid, and 0.5% magnesium stearate;
scheme 2.30: 15% Compound 1, 35% HPMCAS-HG, 4% colloidal silicon dioxide, 35.5% mannitol, 6% CCMC-Na, 4% CMS-Na, and 0.5% magnesium stearate;
scheme 2.31: 12% Compound 1, 28% HPMCAS-HG, 12% Soluplus, 4% colloidal silicon dioxide, 28.5% mannitol, 6% CCMC-Na, 4% CMS-Na, 5% tartaric acid, and 0.5% magnesium stearate;
scheme 2.32: 12% Compound 1, 28% HPMCAS-HG, 12% PVP-VA64, 4% colloidal silicon dioxide, 22.5% mannitol, 6% CCMC-Na, 4% CMS-Na, 10% tartaric acid, 0.5% magnesium stearate, and 1% poloxamer 407;
scheme 2.33: 12% Compound 1, 28% HPMCAS-HG, 12% PVP-VA64, 4% colloidal silicon dioxide, 18.5% mannitol, 6% CCMC-Na, 4% CMS-Na, 10% tartaric acid, 0.5% magnesium stearate, and 5% poloxamer 407;
scheme 2.34: 12% Compound 1, 28% HPMCAS-HG, 12% PVP-VA64, 4% colloidal silicon dioxide, 13.5% mannitol, 6% CCMC-Na, 4% CMS-Na, 10% tartaric acid, 0.5% magnesium stearate, and 10% poloxamer 407.

In some embodiments, the pharmaceutical composition comprises the components in mass percentages according to any one of schemes 2.1 to 2.34.

In some embodiments, in schemes 2.1 to 2.34, Compound 1 and HPMCAS-HG are present as components in the form of a solid dispersion.

In some embodiments, in schemes 2.2 to 2.21, Compound 1 and HPMCAS-HG are present as components in the form of a solid dispersion.

In some embodiments, the pharmaceutical composition preferably comprises the components in mass percentages according to any one of schemes 2.2 to 2.34, and more preferably comprises the components in mass percentages according to any one of schemes 2.3, 2.5, and 2.31.

In some embodiments, the pharmaceutical composition preferably consists of the components in mass percentages according to any one of schemes 2.2 to 2.34, and more preferably consists of the components in mass percentages according to any one of schemes 2.3, 2.5, and 2.31.

The present disclosure further provides a preparation method of the pharmaceutical composition according to any one of the foregoing embodiments of the present disclosure, comprising the step of mixing the components.

In some embodiments, the preparation method of the pharmaceutical composition comprises wet granulation or dry granulation.

In some embodiments, when the pharmaceutical composition is a tablet, the preparation method of the pharmaceutical composition may comprise the steps of:
(1) subjecting the solid dispersion and the solubilizer to wet granulation, wet milling, drying, and dry milling to obtain a granule;
(2) uniformly mixing the granule with the remaining components, followed by tableting to obtain the tablet.

In some embodiments, when the pharmaceutical composition is a tablet, the preparation method of the pharmaceutical composition may comprise the steps of:
(1) subjecting the solid dispersion and the solubilizer to wet granulation, wet milling, drying, and dry milling to obtain a granule;
(2) uniformly mixing the granule with the remaining components, followed by tableting and coating to obtain the tablet.

In some embodiments, when the pharmaceutical composition is a tablet, the preparation method of the pharmaceutical composition may comprise the steps of:
(1) uniformly mixing one or more of the solid dispersion, the solubilizer, the glidant, the filler, the pH adjuster, the disintegrant, and the surfactant, followed by dry granulation and dry milling to obtain a granule;
(2) uniformly mixing the granule with the lubricant and the remaining components, followed by tableting to obtain the tablet.

In some embodiments, when the pharmaceutical composition is a tablet, the preparation method of the pharmaceutical composition may comprise the steps of:
(1) uniformly mixing one or more of the solid dispersion, the solubilizer, the glidant, the filler, the pH adjuster, the disintegrant, and the surfactant, followed by dry granulation and dry milling to obtain a granule;
(2) uniformly mixing the granule with the lubricant and the remaining components, followed by tableting and coating to obtain the tablet.

The present disclosure further provides the use of the solid dispersion according to any one of the foregoing embodiments of the present disclosure in the manufacture of a formulation of Compound 1.

In some embodiments, the formulation of Compound 1 is preferably the pharmaceutical composition according to any one of the foregoing embodiments of the present disclosure.

The present disclosure further provides the use of one or more of the polymeric carrier according to any one of the foregoing embodiments of the present disclosure, the solubilizer according to any one of the foregoing embodiments of the present disclosure, the acidic pH adjuster according to any one of the foregoing embodiments of the present disclosure, the disintegrant according to any one of the foregoing embodiments of the present disclosure, and the surfactant according to any one of the foregoing embodiments of the present disclosure for improving the solubility of Compound 1.

The present disclosure further provides the use of the solid dispersion according to any one of the foregoing embodiments of the present disclosure or the pharmaceutical composition according to any one of the foregoing embodiments of the present disclosure in the manufacture of a medicament.

In one embodiment, the medicament is for the treatment of one or more diseases selected from tumors, pulmonary fibrosis, autoimmune diseases, and neurodegenerative diseases, preferably for the treatment of one or more diseases selected from Alzheimer's disease, amyotrophic lateral sclerosis, multiple sclerosis, stroke, and neuromyelitis optica.

The present disclosure further provides the solid dispersion according to any one of the foregoing embodiments of the present disclosure or the pharmaceutical composition according to any one of the foregoing embodiments of the present disclosure for use in the treatment of one or more diseases selected from tumors, pulmonary fibrosis, autoimmune diseases, and neurodegenerative diseases.

In some embodiments, the solid dispersion or the pharmaceutical composition is preferably for use in the treatment of one or more diseases selected from Alzheimer's disease, amyotrophic lateral sclerosis, multiple sclerosis, stroke, and neuromyelitis optica.

The present disclosure further provides a method of treating a subject in need thereof, comprising administering to the subject an effective amount of the solid dispersion according to any one of the foregoing embodiments of the present disclosure or the pharmaceutical composition according to any one of the foregoing embodiments of the present disclosure; wherein the subject suffers from one or more diseases selected from tumors, pulmonary fibrosis, autoimmune diseases, and neurodegenerative diseases.

In some embodiments, the subject preferably suffers from one or more diseases selected from Alzheimer's disease, amyotrophic lateral sclerosis, multiple sclerosis, stroke, and neuromyelitis optica.

### Definition of Terms

As used herein, the term "solid dispersion (SD)" generally refers to a solid system comprising at least two components, wherein one component is substantially uniformly dispersed throughout the other component. For example, the solid dispersion may be a dispersion of one or more active ingredients (*e.g.*, a drug, such as Compound 1 of the present disclosure) in a polymeric carrier, which may be prepared by melting and/or solvent methods. In the solid dispersion, the active ingredient may exist in a molecular state, colloidal state, metastable state, or amorphous state.

As used herein, the term "drug substance (DS)" refers to an active pharmaceutical ingredient (API) with pharmaceutical activity that has not been formulated into a final drug product, such as the compound itself serving as the active pharmaceutical ingredient.

As used herein, the term "active pharmaceutical ingredient (API)" refers to a compound with pharmaceutical activity, or a pharmaceutically acceptable salt thereof, or mixtures thereof. In the solid dispersion of the present disclosure, the drug substance or active pharmaceutical ingredient is Compound 1 (also sometimes referred to herein directly as API).

As used herein, the term "amorphous solid dispersion (ASD)" refers to a solid dispersion comprising an active pharmaceutical ingredient that is substantially amorphous.

As used herein, the term "amorphous" means that the active ingredient lacks a definite structure, *i.e.*, lacks a crystalline structure.

As used herein, the term "substantially amorphous", "near-amorphous", or "substantially non-crystalline" refers to the absence of a significant amount of crystalline drug, for example, no more than approximately 5% crystallinity, such as no more than approximately 2% or no more than approximately 1% crystallinity, as determined by X-ray diffraction analysis. In one specific embodiment, "substantially non-crystalline" may refer to no detectable crystallinity as determined by one or both of X-ray diffraction analysis or polarized light microscopy.

The term "polymeric carrier", also referred to as "macromolecular carrier", refers to a chain-like or network-like macromolecular compound formed by covalently linked repeating backbone units. In the art, polymeric carriers commonly used in solid dispersions include, but are not limited to, polyethylene glycol (PEG), polyvinylpyrrolidone (PVP), hydroxypropyl methylcellulose (HPMC), hydroxypropyl methylcellulose acetate succinate (HPMCAS), polyacrylic resins, and Soluplus (polyvinyl caprolactam-polyvinyl acetate-polyethylene glycol graft copolymer). For example, one of PEG4000, PEG6000, Eudragit L100-55, Eudragit S100, PVP K17, PVP K30, PVP K90, PVP-VA64, Plasdone S-630, Plasdone S-630 ULTRA, HPMCAS-LF, HPMCAS-MF, HPMCAS-HF, HPMCAS-LG, HPMCAS-MG, HPMCAS-HG, and Soluplus, or any combination thereof may be used as the polymeric carrier in the solid dispersion.

In the art, the solid dispersion may further comprise additional additives as needed, such as lubricants, fillers, disintegrants, and stabilizers (*e.g.*, antioxidants, photostabilizers, free radical scavengers, stabilizers against microbial attack). The specific selection and amounts of such additives are within the routine practice of those skilled in the art.

The solid dispersion of the present disclosure may be prepared using any suitable method, including, but not limited to, solvent-assisted methods such as solvent evaporation and solvent precipitation, as well as solvent-free solid-state methods such as melting and hot-melt extrusion. The solvent evaporation may specifically include film coating, rotary evaporation, freeze-drying, supercritical fluid extraction, or spray drying.

In the solvent evaporation, the active ingredient (*e.g.*, Compound 1 of the present disclosure), the polymeric carrier (*e.g.*, Soluplus), and optionally a surfactant and/or other excipients may be dissolved in a suitable solvent, followed by solvent evaporation to obtain the solid dispersion. The solid obtained after solvent evaporation may be further vacuum-dried to obtain the solid dispersion. It should be understood that the active ingredient, the polymeric carrier, and optionally the surfactant may be dissolved separately in a solvent before mixing, or may be dissolved together in a solvent. When the active ingredient and the polymeric carrier are dissolved together in a solvent, either component may be added first to achieve complete dissolution, followed by addition of the other component. For example, the active ingredient is first dissolved in the solvent, followed by addition of the polymeric carrier; alternatively, the polymeric carrier is first dissolved in the solvent, followed by addition of the active ingredient. When the active ingredient and the polymeric carrier are dissolved separately in a solvent, both components may be dissolved in the same solvent or in different solvents.

The solvent used may be an organic solvent, such as one or more selected from dichloromethane, anhydrous ethanol, methanol, acetone, and ethyl acetate. In some embodiments, the solvent is a mixture of dichloromethane and ethanol. In some embodiments, the weight ratio of dichloromethane to ethanol in the solvent may be approximately 1:1 to approximately 10:1, such as approximately 2:1 to approximately 8:1, approximately 3:1 to approximately 5:1, or approximately 4:1.

It should be understood that while it is desirable to remove as much solvent as possible, a small amount of residual solvent is acceptable. In some embodiments, the residual solvent in the solid dispersion of the present disclosure is no more than approximately 1000 ppm, no more than approximately 900 ppm, no more than approximately 800 ppm, no more than approximately 700 ppm, no more than approximately 600 ppm, no more than approximately 500 ppm, or no more than approximately 400 ppm.

The pharmaceutical composition of the present disclosure may be formulated as solid, semi-solid, liquid, or gaseous dosage forms, such as oral preparations, parenteral preparations, or suppositories. Suitable dosage forms include, but are not limited to, tablets, capsules, granules, powders, suspensions, ointments, solutions, injections, inhalants, and gels. In some embodiments, the pharmaceutical composition of the present disclosure is an enteric-coated preparation, such as an enteric-coated tablet or an enteric-coated capsule (including hard capsules or soft capsules).

The pharmaceutical composition of the present disclosure may be prepared by methods well known in the pharmaceutical field. For example, methods for preparing pharmaceutical compositions are known to those skilled in the art, for example, as described in The Science and Practice of Pharmacy, 20th Edition (Philadelphia College of Pharmacy and Science, 2000).

Routes of administration for the pharmaceutical composition of the present disclosure include, but are not limited to, oral, topical, transdermal, intramuscular, intravenous, inhalation, parenteral, sublingual, rectal, vaginal, and intranasal administration. The pharmaceutical composition of the present disclosure is particularly suitable for transmucosal administration of formulations to a subject, *i.e.*, administration to mucous membranes for transmembrane absorption. Therefore, suitable routes of administration include inhalation, oral, intranasal, and rectal administration, with oral administration being particularly preferred. Dosage forms suitable for oral administration include tablets, capsules, granules, and syrups. However, other routes of administration, such as parenteral administration, are not excluded.

The pharmaceutical composition of the present disclosure may be prepared by combining the solid dispersion of the present disclosure with one or more pharmaceutically acceptable carriers using conventional pharmaceutical methods.

For example, the solid dispersion of the present disclosure may be appropriately processed and mixed with one or more pharmaceutically acceptable carriers to prepare a formulation. The appropriate processing may involve formulating the solid dispersion of the present disclosure into a powder or granule suitable for formulation, and the processing methods include, for example, pulverizing, milling, or grinding.

The term "pharmaceutically acceptable carrier" refers to any carrier included as an inactive ingredient in a pharmaceutical composition, which provides the pharmaceutical composition with an appearance and properties suitable for administration. Pharmaceutically acceptable carriers, such as stabilizers, diluents, additives, adjuvants, and excipients, are substantially free of long-term or permanent adverse effects when administered to a subject. A "pharmaceutically acceptable carrier" should be a pharmaceutically inert material with essentially no biological activity and constitutes the major portion of the formulation.

In the present disclosure, the pharmaceutically acceptable carrier includes, but is not limited to, solubilizers, acidic pH adjusters, glidants, surfactants, disintegrants, diluents, binders, fillers, antioxidants, and lubricants.

In the solid dispersion and pharmaceutical composition of the present disclosure, the mass percentage of each component may vary by approximately ±2%, approximately ±1.5%, approximately ±1%, approximately ±0.5%, approximately ±0.2%, approximately ±0.1%, approximately ±0.05%, approximately ±0.02%, or approximately ±0.01%.

In the present disclosure, the term "approximately" preceding a numerical value indicates that the numerical value may vary by ±2%, ±1.5%, ±1%, ±0.5%, ±0.2%, ±0.1%, ±0.05%, ±0.02%, or ±0.01%.

As used herein, the term "solubilizer" refers to a third substance added to form soluble intermolecular complexes, associations, or double salts with a poorly soluble drug in a solvent, thereby increasing the solubility of the drug in the solvent. In the art, suitable polymeric solubilizers may be, for example, one or more of PEG, PVP, HPMCAS, HPMC, Eudragit, and Soluplus.

As used herein, the term "acidic pH adjuster" refers to an excipient for maintaining or adjusting the acidity or alkalinity of a pharmaceutical product. In the art, suitable acidic pH adjusters may be, for example, one or both of organic acids and inorganic acids. The organic acid is, for example, selected from one or more of citric acid, fumaric acid, malic acid, maleic acid, tartaric acid, succinic acid, oxalic acid, aspartic acid, mandelic acid, glutaric acid, and glutamic acid. The inorganic acid is, for example, selected from one or more of hydrochloric acid, phosphoric acid, nitric acid, and sulfuric acid.

As used herein, the term "glidant" refers to an excipient that can reduce interparticle friction and improve the flowability of powders (granules). In the art, the glidant may be, for example, selected from one or more of colloidal silicon dioxide, animal or vegetable fats, and waxes.

As used herein, the term "surfactant" refers to a class of compounds containing fixed hydrophilic and lipophilic groups, which, due to their amphiphilic nature, tend to concentrate at the surface of a solution, at the interface between two immiscible liquids, or at the interface between a liquid and a solid, thereby reducing surface tension or interfacial tension. In the art, surfactants may generally further enhance the therapeutic potential of solid dispersions, and may include, for example, anionic surfactants, such as sodium dodecyl sulfate (sodium lauryl sulfate) and docusate sodium; cationic surfactants, such as cetrimonium bromide, benzethonium chloride, cetylpyridinium chloride, and lauric acid; nonionic surfactants, such as polyoxyethylene alkyl ethers, polyoxyethylene sorbitan fatty acid esters (*e.g.*, Tween 80, 60, 40, and 20), polyoxyethylene castor oil derivatives (*e.g.*, Cremophor RH40), polyoxyethylene stearates, vitamin E polyethylene glycol succinate, and poloxamers (poloxamer 188 and poloxamer 407).

As used herein, the term "disintegrant" refers to an ingredient added to a formulation to promote rapid disintegration into small units and enable faster drug dissolution. In the art, suitable disintegrants may be, for example, one or more of povidone, crospovidone, carboxymethyl cellulose, methylcellulose, alginic acid, CCMC-Na, CMS-Na, starches (*e.g.*, corn starch, potato starch), formaldehyde-casein, alginic acid, silica, and guar gum.

As used herein, the term "diluent", also referred to as "filler", refers to an ingredient for increasing the volume or weight of a formulation. Diluents typically constitute a large proportion of pharmaceutical dosage forms, functioning not only to ensure a certain size of the dosage form but also to reduce dosage variations of the active pharmaceutical ingredient and improve the compressibility of the drug. In the art, suitable diluents may be, for example, one or more of sugars (*e.g.*, maltose, lactose, fructose, sucrose), cellulose materials (*e.g.*, microcrystalline cellulose, silicified microcrystalline cellulose, powdered cellulose), starches (*e.g.*, pregelatinized corn starch, maltodextrin, dextrin), sugar alcohols (*e.g.*, mannitol, sorbitol), dextrates, calcium phosphate, gums, acrylates (*e.g.*, polymethacrylates), calcium carbonate, magnesium oxide, and talc.

As used herein, the term "binder" refers to a class of solid powders or solutions with adhesive properties that aggregate non-adhesive or insufficiently adhesive powders into granules and facilitate compression molding. In the art, suitable binders may be, for example, one or more of gum arabic, gelatin, carbomer, dextrin, starch (*e.g.*, corn starch), povidone, copovidone, carboxymethyl cellulose, guar gum, glucose, hydroxypropyl cellulose, hydroxypropyl methylcellulose, methylcellulose, ethylcellulose, hydroxypropyl methylcellulose acetate succinate, polymethacrylate, maltodextrin, and hydroxyethyl cellulose.

As used herein, the term "antioxidant" refers to a class of substances capable of effectively preventing or delaying auto-oxidation. As a crucial component of pharmaceutical excipients, antioxidants are primarily used for preventing oxidative degradation of drugs and their formulations, as well as discoloration, precipitation, and other instabilities caused by oxidation. In the art, suitable antioxidants may be, for example, ascorbic acid or sodium ascorbate.

As used herein, the term "lubricant" refers to a substance that reduces friction between tablets and die walls. In the art, suitable lubricants may be, for example, one or more of polyethylene glycol (*e.g.*, with a molecular weight of 1000 to 6000), magnesium stearate, calcium stearate, and sodium stearyl fumarate.

As used herein, the term "glidant" refers to a substance that reduces friction between particles, thereby improving powder flowability. In the art, suitable glidants may be, for example, selected from one or more of colloidal silicon dioxide, animal or vegetable fats, and waxes.

As used herein, the term "plasticizer" refers to a low molecular weight substance that, when added to another material (typically a polymer), provides the polymeric material with flexibility and elasticity as well as ease of processing. In the art, plasticizers generally improve the processability of the solid dispersion and may be, for example, selected from one or more of acetyl tributyl citrate, acetyl triethyl citrate, benzyl benzoate, chlorobutanol, dextrin, dibutyl phthalate, diethyl phthalate, dimethyl phthalate, glycerol, glyceryl monostearate, mannitol, mineral oil, lanolin alcohol, palmitic acid, polyethylene glycol, polyvinyl acetate phthalate, propylene glycol, 2-pyrrolidone, sorbitol, stearic acid, triacetin, tributyl citrate, triethanolamine, and triethyl citrate.

As used herein, the term "HPMCAS" refers to hydroxypropyl methylcellulose acetate succinate, which may be, for example, HPMCAS-LF, HPMCAS-LG, HPMCAS-MF, HPMCAS-MG, HPMCAS-HF, or HPMCAS-HG.

As used herein, the term "PVP-VA" refers to vinylpyrrolidone-vinyl acetate copolymer (copovidone), which may be, for example, PVP-VA64, PVP-VA37, or PVP-VA73.

As used herein, the term "PEG" refers to polyethylene glycol, which may be, for example, PEG4000 or PEG6000.

As used herein, the term "PVP" refers to polyvinylpyrrolidone (povidone), which may be, for example, PVP K17, PVP K30, PVP K90, PVP-VA64, Plasdone S-630, or Plasdone S-630 ULTRA.

As used herein, the term "HPMC" refers to hydroxypropyl methylcellulose, which may be, for example, HPMC E3LV, E5LV, E6LV, E15LV, E50LV, E4M, E10M, K100LV, K4M, K15M, L100M, A15LV, A4C, A15C, A4M, and other related grades.

As used herein, the term "Eudragit" refers to polyacrylic resin, which may be, for example, Eudragit L100-55 or Eudragit S100.

As used herein, the term "Soluplus" refers to polyvinyl caprolactam-polyvinyl acetate-polyethylene glycol graft copolymer.

The solid dispersion or pharmaceutical composition of the present disclosure may comprise a film coating. The film coating may improve taste and provide an elegant appearance. If desired, the film coating may be an enteric coating. The film coating typically comprises a film-forming polymer, such as hydroxypropyl methylcellulose, hydroxypropyl cellulose, and acrylate or methacrylate copolymers. In addition to the film-forming polymer, the film coating may further comprise a plasticizer such as polyethylene glycol, a surfactant such as Tween series, and optionally a pigment such as titanium dioxide or iron oxide. The film coating may further comprise talc as an antiadherent. The film coating of tablets may also improve swallowability.

Enteric-coated preparations are typically prepared by film coating, which generally involves depositing a film layer of an acid-insoluble (enteric-coated) polymer onto the surface of a pre-prepared dosage form, for example, by spraying an aqueous or organic solution or suspension of the enteric-coated polymer onto tumbling or moving tablets or capsules, followed by drying with hot air to deposit the enteric-coated polymer.

The solid dispersion or pharmaceutical composition of the present disclosure may be provided in a unit dosage form containing a predetermined amount of active ingredient per unit dose. In the solid dosage form of the pharmaceutical composition of the present disclosure, the dose of active ingredient may be 0.1 to 100 mg per unit dose or 10 to 20 mg per unit dose.

The solid dispersion or pharmaceutical composition of the present disclosure may be administered in a therapeutically effective amount to a subject in need thereof. As used herein, the term "therapeutically effective amount" refers to an amount that achieves the desired therapeutic effect within a specific dosage range and time period. The "therapeutically effective amount" is determined by factors such as the specific condition being treated, the individual being treated, the cause of the condition, the target of the drug, and the mode of administration.

For parenteral administration, the dose may be 0.1 to 200 mg of active ingredient/kg body weight/day. For oral administration, the dose may be 0.1 to 1000 mg of active ingredient/kg body weight/day, such as 0.1 to 100 mg of active ingredient/kg body weight/day.

As used herein, the term "solubility" refers to the solubility of the active ingredient in aqueous media, such as water, buffers, simulated gastric or intestinal fluid (*e.g.*, FaSSIF, FeSSIF, or SGF), gastric fluid, or intestinal fluid.

As used herein, the term "bioavailability" refers to the extent to which a drug is utilized by target tissues (*e.g.*, the intestine in the present disclosure) after administration. For example, "bioavailability" may refer to the fraction of the drug absorbed after administration to a subject in a fed or fasted state.

In certain aspects, when the solid dispersion or pharmaceutical composition of the present disclosure is administered to a subject in a fasted or fed state, the bioavailability of Compound 1 is at least approximately 15% of the administered dose, but may be greater than 20%, 25%, 30%, 35%, 40%, 45%, 50%, 60%, or 70%.

As used herein, the term "fasted" may also be referred to as "fasting state", and generally refers to a period of at least 2 hours before eating by a subject and at least 2 hours after the previous meal.

As used herein, the term "fed" may also be referred to as "postprandial", and generally refers to a period within 2 hours after eating by a subject.

The solid dispersion or pharmaceutical composition of the present disclosure may be used for treating diseases mediated by CSF-1R kinase.

The diseases mediated by CSF-1R kinase may be selected from multiple myeloma, acute myeloid leukemia (AML), chronic myeloid leukemia (CML), prostate cancer, breast cancer, ovarian cancer, melanoma, glioblastoma multiforme, giant cell tumor of bone, non-small cell lung cancer, tenosynovial giant cell tumor, tumor metastasis, myelofibrosis, pigmented villonodular synovitis, and gastrointestinal stromal tumor.

Based on common knowledge in the art, the above preferred conditions may be combined arbitrarily to obtain various preferred embodiments of the present disclosure.

All reagents and raw materials used in the present disclosure are commercially available.

The English terms or abbreviations involved in the present disclosure and their corresponding full names are listed below:

| English abbreviation | Full name | English abbreviation | Full name |
|---|---|---|---|
| HPMCAS | Hydroxypropyl methylcellulose acetate succinate | API | Active pharmaceutical ingredient |
| Soluplus | Polyvinyl caprolactam-polyvinyl acetate-polyethylene glycol graft copolymer | FaSSIF | Fasted state simulated intestinal fluid |
| PEG | Polyethylene glycol | FeSSIF | Fed state simulated intestinal fluid |
| PVP | Polyvinylpyrrolidone | SGF | Simulated gastric fluid |
| Eudragit | Polyacrylic resin | AUC | Area under the curve |
| HPMC | Hydroxypropyl methylcellulose | Cₘₐₓ | Peak plasma concentration |
| CCMC-Na | Croscarmellose sodium | Cremophor | Polyoxyl hydrogenated castor oil |
| CMS-Na | Sodium starch glycolate | XRPD | X-ray powder diffraction |
| CMC-Na | Sodium carboxymethyl cellulose | SDS | Sodium dodecyl sulfate |

The positive and progressive effects of the present disclosure are as follows:
(1) The solid dispersion prepared from the composition of the present disclosure is more conducive to dispersing Compound 1 in the polymeric carrier in an amorphous (or near-amorphous) or non-crystalline state. Furthermore, the solid dispersion prepared from the composition of the present disclosure is conducive to improving the solubility of Compound 1.
(2) Formulating Compound 1 into a solid dispersion can significantly improve its solubility and bioavailability. In particular, Soluplus, Plasdone S-630 ULTRA, or HPMCAS-HG exhibits a better solubilizing effect on Compound 1 compared to other polymeric carriers (*e.g.*, PEG4000, PEG6000, Eudragit L100-55, Eudragit S100, PVP K17, PVP K30, PVP K90, Plasdone S-630, HPMCAS-LF, HPMCAS-MF, HPMCAS-HF). The solubility of the solid dispersion of Compound 1 prepared using Soluplus or Plasdone S-630 ULTRA as the polymeric carrier is higher than that prepared using other polymeric carriers (*e.g.*, PEG4000, PEG6000, Eudragit L100-55, Eudragit S100, PVP K17, PVP K30, PVP K90, Plasdone S-630, HPMCAS-LF, HPMCAS-MF, HPMCAS-HF). Under intestinal and/or gastric fluid conditions, the solubility of the solid dispersion of Compound 1 prepared using Soluplus, Plasdone S-630 ULTRA, or HPMCAS-HG is significantly improved compared to Compound 1 itself, and is superior to that prepared using other polymeric carriers (*e.g.*, PEG4000, PEG6000, Eudragit L100-55, Eudragit S100, PVP K17, PVP K30, PVP K90, Plasdone S-630, HPMCAS-LF, HPMCAS-MF, HPMCAS-HF).
(3) The pharmaceutical composition of the present disclosure provides one or more of the advantages of improved pharmacokinetics, increased *in vivo* exposure, and enhanced solubility. Furthermore, the increased *in vivo* exposure reduces the dosage of Compound 1, which can significantly lower production costs during drug development, for example, by reducing tablet size or formulating as a dry suspension, and can thus improve patient compliance. Accordingly, the pharmaceutical composition of the present disclosure further provides one or both of the advantages of low production cost and good patient compliance.

### BRIEF DESCRIPTION OF THE DRAWINGS

FIG. 1 shows the XRPD results after 2 hours in different media for solid dispersions prepared by spray drying using different polymeric carriers; (a) XRPD results of API after 2 hours; (b) XRPD results of API + HPMCAS-HG after 2 hours; (c) XRPD results of API + Soluplus after 2 hours; (d) XRPD results of API + Plasdone S-630 ULTRA after 2 hours; from top to bottom: XRPD before solubility testing, after 2 hours in FaSSiF medium, after 2 hours in FeSSiF medium, and after 2 hours in SGF medium.
FIG. 2 shows the absorption of the solid dispersion prepared using HPMCAS-HG as the polymeric carrier in rats under different routes of administration; in the bar charts of AUC and Cₘₐₓ shown in the lower panel, the left side represents the gastric model and the right side represents the intestinal model.
FIG. 3 shows the effect of different polymeric carriers on absorption in a rat gastric model; the three samples, from left to right, are solid dispersions prepared using HPMCAS-HG, Soluplus, and Plasdone S-630 ULTRA as polymeric carriers.
FIG. 4 shows a comparison of *in vivo* exposure in dogs.
FIG. 5 shows a comparison of dissolution profiles in pH 4.5 medium for different concentrations of acidic pH adjusters.
FIG. 6 shows a comparison of two-stage dissolution profiles for different concentrations of acidic pH adjusters.
FIG. 7 shows a comparison of dissolution profiles in pH 4.5 medium for different types of acidic pH adjusters.
FIG. 8 shows a comparison of two-stage dissolution profiles for different types of acidic pH adjusters.
FIG. 9 shows a comparison of dissolution profiles in pH 4.5 medium for different types of solubilizers.
FIG. 10 shows a comparison of two-stage dissolution profiles for different types of solubilizers.
FIG. 11 shows a comparison of dissolution profiles in pH 4.5 medium for different concentrations of solubilizers.
FIG. 12 shows a comparison of two-stage dissolution profiles for different concentrations of solubilizers.

### DETAILED DESCRIPTION OF THE PREFERRED EMBODIMENT

The present disclosure is further illustrated below by way of examples, but is not thereby limited to the scope of the examples provided. In the following examples, experimental methods without specified conditions are selected according to conventional methods and conditions, or according to product instructions.

The following reagents and analytical methods will be used in the examples of the present disclosure: 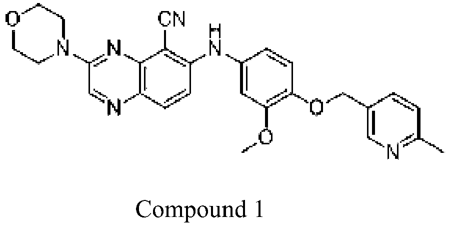
Chemical name: 6-((3-methoxy-4-((6-methylpyridin-3-yl)methoxy)phenyl)amino)-3-morpholinoquinoxaline-5-carbonitrile;
Molecular formula: C₂₇H₂₆N₆O₃.

### 1. Reagents

(1) Compound 1, obtained from Asymchem Laboratories (Tianjin) Co., Ltd.
(2) PEG6000, obtained from Jiangxi Alpha Hi-Tech Pharmaceutical Co., Ltd.
(3) Soluplus, obtained from BASF.
(4) Plasdone S-630 ULTRA, obtained from Ashland.
(5) HPMCAS-HG, obtained from Shin-Etsu, viscosity: 3 mPa·s.
(6) PVP K30, obtained from BASF.
(7) PVP-VA64, obtained from BASF.
(8) HPMC E3LV, obtained from Colorcon.
(9) Anhydrous ethanol and dichloromethane, both obtained from Tianjin Tairong Chemical Trading Co., Ltd.
(10) Fumaric acid, obtained from Finar.
(11) Malic acid, obtained from Alpha.
(12) Fumaric acid, obtained from Finar.
(13) Tartaric acid, obtained from Hunan Er-Kang Pharmaceutical Co., Ltd.

### 2. PLM method:

PLM images are collected using a Leica DM750P polarized light microscope (Leica, Germany). A small amount of each sample is placed on a glass slide, followed by addition of a drop of cedarwood oil, and the slide is covered with a cover slip. The samples are observed using polarized light at an appropriate magnification. The instrument control software is LAS (V4.9), and data analysis is performed using LAS (V4.9).

### 3. XRPD method:

The samples are analyzed for their crystal forms using a D8 FOCUS X-ray powder diffractometer (Bruker, Germany) in reflection mode. The samples are scanned at a 2θ angle of 3° to 42°, a step size of 0.02°, a scan time of 0.2 seconds per step, and a temperature of 25°C. The X-ray tube voltage and current are 40 kV and 40 mA, respectively. For sample preparation, an appropriate amount of sample is placed on a polished sample holder and gently flattened using a spatula or glass slide to ensure a smooth and flat surface. XRPD patterns are analyzed using DIFFRAC.EVA (V4.3.1).

### 4. Residual solvent and purity analysis:

A 50 mg/mL test solution is prepared using 0.25 mg/mL anhydrous ethanol solution and 0.03 mg/mL dichloromethane solution as reference solutions, and 0.01 mg/mL anhydrous ethanol solution and 0.006 mg/mL dichloromethane solution as sensitivity solutions, with DMSO as the solvent for all the above solutions. Analysis is performed using gas chromatography.

### 5. Solubility testing method:

Preparation of FaSSIF stock solution with pH buffer: Approximately 621 mg of NaCl, approximately 345 mg of NaH₂PO₄, and approximately 41.9 mg of NaOH are weighed into a 100 mL volumetric flask, and dissolved in water to approximately 5 cm below the mark. After cooling and pH adjustment, the resulting mixture is diluted to volume with water, and mixed thoroughly with shaking.

FeSSIF stock solution: Approximately 866 mg of glacial acetic acid, approximately 406 mg of NaOH, and approximately 1184 mg of NaCl are weighed into a 100 mL volumetric flask, and dissolved in water to approximately 5 cm below the mark. After cooling and pH adjustment, the resulting mixture is diluted to volume with water, and mixed thoroughly with shaking.

SGF solution: Approximately 202 mg of NaCl and approximately 321 mg of pepsin are weighed into a 100 mL volumetric flask, followed by addition of 700 µL of HCl, and dissolved in water to approximately 5 cm below the mark. After cooling, the resulting mixture is diluted to volume with water, and mixed thoroughly with shaking.

FaSSIF solution: FaSSIF stock solution is transferred to a 100 mL volumetric flask, followed by addition of approximately 225 mg of FaSSIF powder, and dissolved in FaSSIF stock solution to approximately 5 cm below the mark. After cooling, the resulting mixture is diluted to volume with FaSSIF stock solution, mixed thoroughly with shaking, and allowed to stand for 2 hours before use.

FeSSIF solution: FeSSIF stock solution is transferred to a 100 mL volumetric flask, followed by addition of approximately 1122 mg of FeSSIF powder, and dissolved in FeSSIF stock solution to approximately 5 cm below the mark. After cooling, the resulting mixture is diluted to volume with FeSSIF stock solution, and mixed thoroughly with shaking.

### Experimental procedure

Approximately 20 mg of sample is weighed into a 4 mL vial, followed by addition of 4 mL of different solvents (FaSSIF, FeSSIF, SGF). After the vial is equipped with a magnetic stir bar, the mixture is stirred at 37°C for 2 hours, during which samples are taken at 0.5 hours and 2 hours. After filtration, the sample concentration in the filtrate is determined by HPLC. The pH of the 2-hour filtrate and the crystal form of the remaining solid are also measured.

### Example 1: Solid dispersion

### 1.1 Small-scale rotary evaporation experiment 1

Formulation information is shown in Table 1.

**Table 1:**

| **Material name** | | **Mass percentage wt/wt (%)** | | | **Formulation amount (g)** |
|---|---|---|---|---|---|
| | | **Formulation 1** | **Formulation 2** | **Formulation 3** | |
| API | Compound 1 | 25.0 | 25.0 | 25.0 | 1.5 |
| Polymeric carrier | PEG6000 | 75.0 | -- | -- | 4.5 |
| | Soluplus | -- | 75.0 | -- | 4.5 |
| | Plasdone^{™} S-630 ULTRA | -- | -- | 75.0 | 4.5 |
| Solvent^{a} | Dichloromethane | N/A | N/A | N/A | 45 |
| | Ethanol | N/A | N/A | N/A | 15 |

| | | | | | |
|---|---|---|---|---|---|
| Note: a indicates the solvent is removed during sample preparation. | | | | | |

Process parameters are shown in Table 2.

**Table 2:**

| **Process** | **Equipment** | **Parameter** | **Observation** |
|---|---|---|---|
| Weighing | Electronic balance | Weighing the API, carrier, and solvent according to the formulation amount | Smooth process |
| Solution preparation | Beaker and stir bar | Transferring the carrier to a beaker containing a mixed solvent of dichloromethane and ethanol and stirring until completely dissolved, followed by addition of the API and stirring until completely dissolved, resulting in a clear solution | Clear solution |
| Rotary evaporation | Rotary evaporator and circulating water multi-purpose vacuum pump | Transferring the solution to a round-bottom flask, then removing the solvent by rotary evaporation at 50°C using a rotary evaporator, and scraping the resulting sample from the round-bottom flask | Smooth process |
| Vacuum drying | Vacuum oven | Vacuum drying at 50°C and -0.08 to -0.085 MPa for 4 hours | Smooth process |

### Analysis results:

Using the rotary evaporation method with an API-to-carrier ratio of 1:3, none of the prepared samples were amorphous, and PEG6000 exhibited the poorest crystallization inhibitory effect.

### 1.2 Small-scale rotary evaporation experiment 2

Formulation information is shown in Table 3.

**Table 3:**

| **Material name** | | **Mass percentage wt/wt (%)** | | **Formulation amount (g)** |
|---|---|---|---|---|
| | | **Formulation 4** | **Formulation 5** | |
| API | Compound 1 | 20.0 | 25.0 | 0.5 |
| Polymeric carrier | Soluplus | 80.0 | -- | 2.0 |
| | HPMCAS-HG | -- | 75.0 | 1.5 |
| Solvent^{a} | Dichloromethane | N/A | N/A | 37.5 |
| | Ethanol | N/A | N/A | 12.5 |

| | | | | |
|---|---|---|---|---|
| Note: a indicates that the solvent is removed during sample preparation. | | | | |

Process parameters are shown in Table 4.

**Table 4:**

| **Process** | **Solid dosage form R&D laboratory** | | |
|---|---|---|---|
| | **Equipment** | **Parameter** | **Observation** |
| Weighing | Electronic balance | Weighing the API, carrier, and solvent according to the formulation amount | Smooth process |
| Solution preparation | Beaker and stir bar | Transferring the carrier to a beaker containing a mixed solvent of dichloromethane and ethanol and stirring until completely dissolved, followed by addition of the API and stirring until completely dissolved, resulting in a clear solution | Clear solution |
| Rotary evaporation | Rotary evaporator and circulating water multi-purpose vacuum pump | Transferring the solution to a round-bottom flask, then removing the solvent by rotary evaporation at 50°C using a rotary evaporator, and scraping the resulting sample from the round-bottom flask | Smooth process |
| Vacuum drying | Vacuum oven | Vacuum drying at 50°C and -0.08 to -0.085 MPa for 4 hours | Smooth process |

### Analysis results:

Using the rotary evaporation method with an API-to-HPMCAS-HG ratio of 1:3 and an API-to-Soluplus ratio of 1:4, none of the prepared samples were completely amorphous, demonstrating that the rotary evaporation method cannot produce amorphous solid dispersions.

### 1.3 Spray drying test

Formulation information is shown in Table 5.

**Table 5:**

| **Material name** | | **Mass percentage wt/wt (%)** | | | **Formulation amount (g)** |
|---|---|---|---|---|---|
| | | **Formulation 6** | **Formulation 7** | **Formulation 8** | |
| API | Compound 1 | 25.0 | 25.0 | 30.0 | 1.5 |
| Polymeric carrier | Soluplus | 75.0 | -- | -- | 4.5 |
| | Plasdone S-630 ULTRA | -- | 75.0 | -- | 4.5 |
| | HPMCAS-HG | -- | -- | 70.0 | 3.5 |
| Solvent^{a} | Dichloromethane | N/A | N/A | | 96.0 |
| | Ethanol | N/A | N/A | | 24.0 |

| | | | | | |
|---|---|---|---|---|---|
| Note: a indicates that the solvent is removed during sample preparation. | | | | | |

### Preparation process of Formulation 8:

Solution preparation: Dichloromethane and anhydrous ethanol were weighed into a solution preparation system. HPMCAS-HG was slowly added to the solution preparation system, followed by stirring until completely dissolved. Compound 1 was then slowly added to the solution preparation system. After the addition was completed, the addition funnel was rinsed with a mixed solvent of dichloromethane and anhydrous ethanol to ensure complete addition of Compound 1, followed by stirring until completely dissolved.

Spray drying: Nitrogen purging and preheating were performed. Parameters were stabilized using blank solvent. After the inlet air temperature reached 70°C ± 5°C and the outlet air temperature reached 45.0°C ± 5.0°C, the feed was switched to the drug solution and spray drying was started.

Double-cone drying: The solid dispersion prepared by spray drying was dried using a double-cone dryer until residual solvents met specifications. In-process samples were taken for content and moisture testing.

Process parameters for Formulas 6 and 7 are shown in Table 6.

**Table 6:**

| **Process** | **OSD1 workshop** | | |
|---|---|---|---|
| | **Equipment** | **Parameter** | **Observation** |
| Weighing | Negative pressure weighing hood and electronic balance | Weighing the API, carrier, and solvent according to the formulation amount | Smooth process |
| Solution preparation | Beaker and stir bar | Transferring the carrier to a beaker containing a mixed solvent of dichloromethane and ethanol and stirring until completely dissolved, followed by addition of the API and stirring until completely dissolved, resulting in a clear solution | Clear solution |
| Spray drying | Spray dryer | Inlet air temperature: 80 ± 5°C | Smooth process |
| | | Outlet air temperature: 65 ± 5°C | |
| | | Peristaltic pump power: 10 to 15% | |
| | | Condensation temperature: -20°C | |
| | | N₂ flow: 30 mm | |
| | | Negative pressure: -25 mbar | |
| Vacuum drying | Vacuum oven | Vacuum drying at 50°C and -0.08 to -0.085 MPa for 4 hours | Smooth process |

Analysis results: Amorphous solid dispersions were successfully prepared by spray drying. The polymeric carriers HPMCAS-HG, Soluplus, and Plasdone^{TM} S-630 ULTRA exhibited superior crystallization inhibitory effects. Residual solvent and purity results are shown in Table 7.

**Table 7:**

| Sample | Residual solvent | Purity |
|---|---|---|
| Formulation 6 (API + Soluplus) | Anhydrous ethanol: 31 ppm | 99.89% |
| | Dichloromethane: 521 ppm | |
| Formulation 7 (API + Plasdone S-630 ULTRA) | Anhydrous ethanol: 69 ppm | 99.91% |
| | Dichloromethane: 488 ppm | |

Solubility results are shown in Table 8.

**Table 8:**

| Sample | Medium | Solubility (mg/mL) | | | |
|---|---|---|---|---|---|
| | | 0.5 h | 2 h | pH after 2 h | pH after 2 h |
| API | FaSSIF | 0.00026 | 0.00026 | 6.58 | N/A |
| | FeSSIF | 0.00313 | 0.00293 | 5.02 | N/A |
| | SGF | 0.6986 | 0.5240 | 1.28 | N/A |
| Formulation 8 (API + HPMCAS-HG) | FaSSIF | 0.00719 | 0.00258 | 5.97 | Near-amorphous |
| | FeSSIF | 0.02291 | 0.00601 | 4.97 | Near-amorphous |
| | SGF | 0.4818 | 0.3532 | 1.25 | Near-amorphous |
| Formulation 6 (API + Soluplus) | FaSSIF | 0.11913 | 0.09093 | 6.43 | Near-amorphous |
| | FeSSIF | 0.12537 | 0.09308 | 5.00 | Near-amorphous |
| | SGF | 1.6528 | 0.9672 | 1.21 | Near-amorphous |
| Formulation 7 (API + Plasdone^{TM} S-630 ULTRA) | FaSSIF | 0.00339 | 0.00232 | 6.45 | Amorphous-to-crystalline transformation |
| | FeSSIF | 0.03210 | 0.00887 | 5.02 | Amorphous-to-crystalline transformation |
| | SGF | 2.5502 | 1.2024 | 1.36 | Near-amorphous |

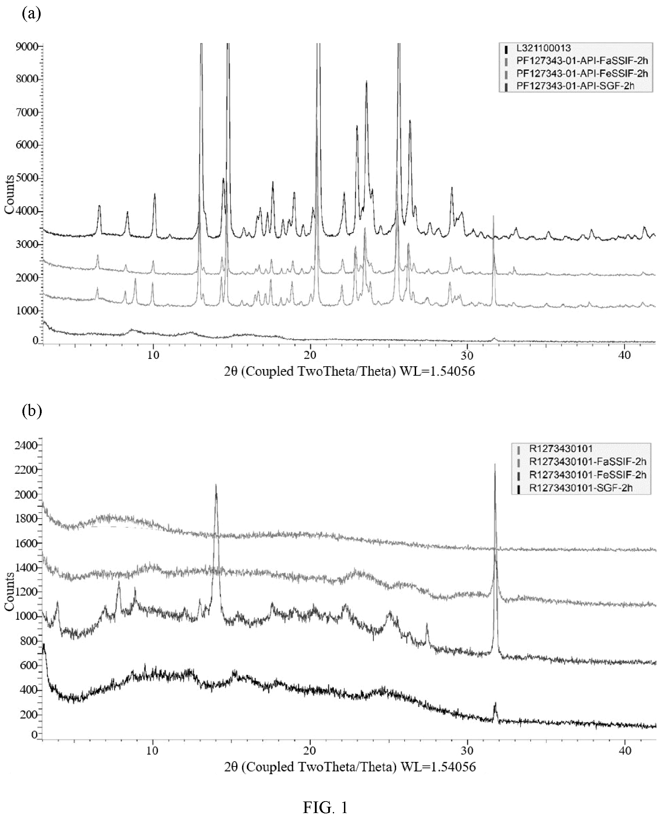

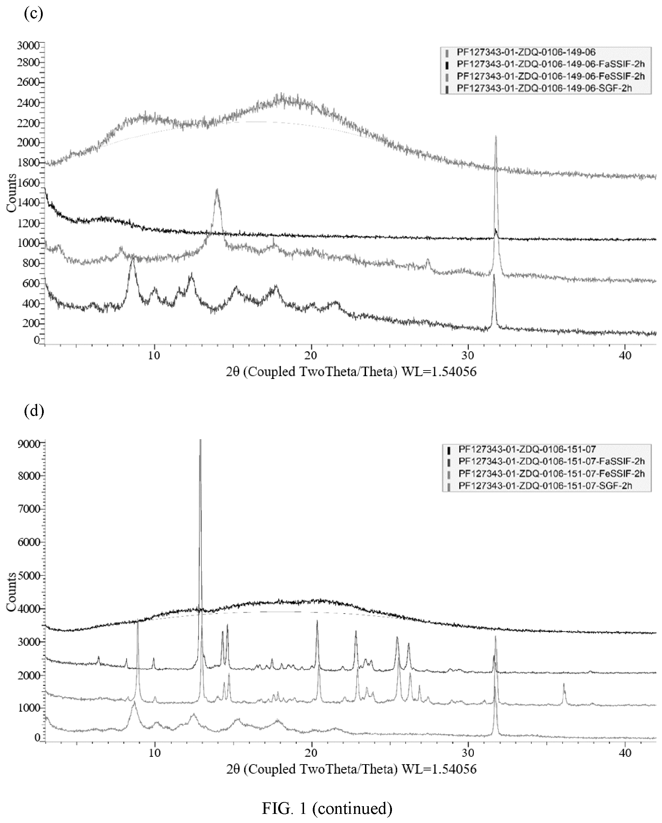

The XRPD results after 2 hours are shown in FIG. 1, wherein (a) shows the XRPD results of API (L321100013) after 2 hours; (b) shows the XRPD results of API + HPMCAS-HG (R1273430101) after 2 hours; (c) shows the XRPD results of API + Soluplus (PF127343-01-ZDQ-0103-149-06) after 2 hours; (d) shows the XRPD results of API + Plasdone S-630 ULTRA (PF127343-01-ZDQ-0106-151-07) after 2 hours; from top to bottom: XRPD before solubility testing, after 2 hours in FaSSIF medium, after 2 hours in FeSSIF medium, and after 2 hours in SGF medium.

The solubility results demonstrated that under intestinal and gastric fluid conditions, the solubility of samples using Soluplus or Plasdone S-630 ULTRA as the carrier was significantly improved compared to the API; under intestinal fluid conditions, the solubility of samples using HPMCAS-HG as the carrier was significantly improved compared to the API, and samples using Soluplus as the carrier exhibited more stable crystal forms.

### 1.4 PK study in rats

### Experimental methods:

1) Nine SD rats, 3 per group, SPF grade, adult males, 6 to 8 weeks old, weighing 280 to 320 g at the start of the experiment.
2) Formulations: API: Soluplus = 1:3 (Formulation 6), API: Plasdone^{TM} S-630 ULTRA = 1:3 (Formulation 7), API: HPMCAS-HG = 3:7 (Formulation 8).
3) Vehicle: 0.5% MC (1500 cp), pH 1.2.
4) Dose: 60 mg/kg based on API.
5) Dosing volume: 10 mL/kg.
6) Route of administration: single oral dose; fasting overnight for 12 hours before administration and resuming feeding 2 hours after administration, with free access to water.
7) Blood sampling points: 0.25 h, 0.5 h, 1 h, 2 h, 4 h, 6 h, 8 h, 10 h, and 24 h.
8) Sample collection: 100 µL of blood was collected into an anticoagulant tube containing EDTA-2K, which was gently inverted to ensure thorough mixing of the anticoagulant (EDTA-2K) with the blood, then stored in an ice-water bath, and centrifuged at 4°C and 8000 rpm for 5 minutes within 1 to 2 hours after blood collection. The plasma obtained after centrifugation was transferred to a labeled EP tube and rapidly stored in an ultra-low temperature freezer at -80°C until sample analysis.
9) Experimental results:

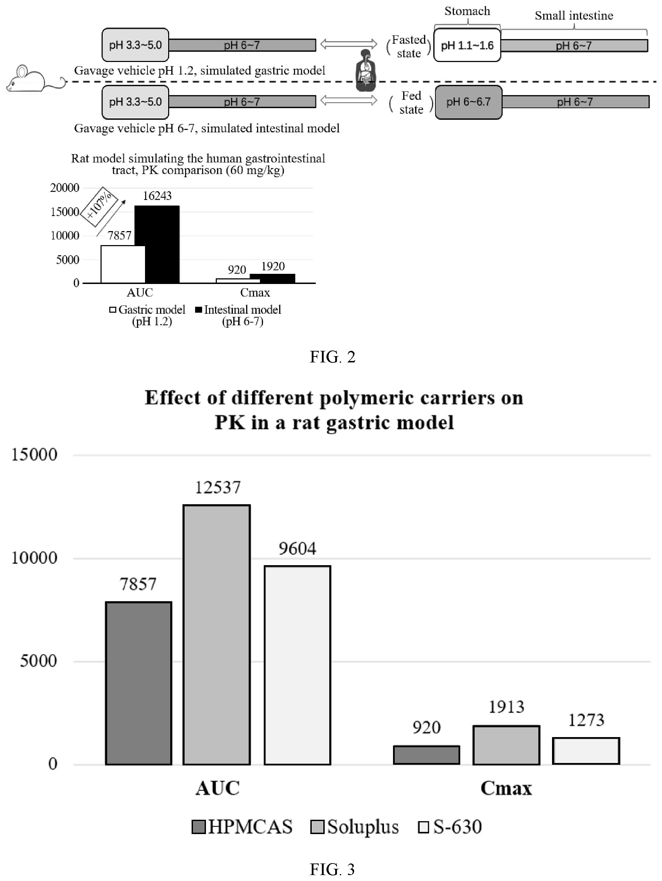

Referring to FIG. 2, the reference solid dispersion was administered to rats via different routes (gastric model (pH 1.2) and intestinal model (pH 6 to 7)) to simulate gastric (preprandial) and intestinal (postprandial) dissolution in humans. For the gastric model (pH 1.2), rats were administered a hydrochloric acid solution (pH 1.2) containing 5% MC via gavage to lower the pH. For the intestinal model (pH 6 to 7), rats were administered an aqueous solution containing 5% MC via gavage, and no increase in pH was observed in the gastrointestinal tract. Drug exposure in the intestinal model was found to be greater than that in the gastric model, with AUC increased by 107%. The results indicate that evaluating solid dispersions in the gastric model better reflects the actual situation in humans, while also suggesting that changes in gastrointestinal pH in rats affect the absorption of the reference solid dispersion.

Referring to FIG. 3, since the gastrointestinal pH in rats is relatively high, typically greater than 3, all three solid dispersions were administered to rats via gavage using a hydrochloric acid solution (pH 1.2) containing 5% MC to lower the pH and simulate changes in gastrointestinal pH in humans. Based on the results from the PK study in rats, Formulas 6, 7, and 8 exhibited good *in vivo* exposure in rats, indicating that the polymeric carriers in the solid dispersions can enhance the tolerance of formulations to changes in gastrointestinal pH and improve *in vivo* absorption.

### 1.5 Solid dispersion for subsequent examples

### Formulation:

| Component | Active pharmaceutical ingredient (Compound 1) | HPMCAS | Dichloromethane* | Anhydrous ethanol* |
|---|---|---|---|---|
| Mass percentage | 30% | 70% | Removed during spray drying | |

| | | | | |
|---|---|---|---|---|
| Note: * indicates that the solvent is removed during sample preparation. | | | | |

### Preparation process:

1) Solution preparation: Dichloromethane and anhydrous ethanol were weighed into a solution preparation system. HPMCAS was slowly added to the solution preparation system, followed by stirring until completely dissolved. Compound 1 was then slowly added to the solution preparation system. After the addition was completed, the addition funnel was rinsed with a mixed solvent of dichloromethane and anhydrous ethanol to ensure complete addition of Compound 1, followed by stirring until completely dissolved.
2) Spray drying:

Nitrogen purging and preheating were performed.

Spray drying: Parameters were stabilized using blank solvent. After the inlet air temperature reached 70°C ± 5°C and the outlet air temperature reached 45.0°C ± 5.0°C, the feed was switched to the drug solution and spray drying was started.

Double-cone drying: The solid dispersion prepared by spray drying was dried using a double-cone dryer until residual solvents met specifications. In-process samples were taken for content and moisture testing.

3) The prepared solid dispersion was used for the preparation of formulations in subsequent examples.

### Example 2: Tablet

### 2.1 Tablet formulation:

| Component | Solid dispersion | Povidone K90 (intragranular) | Povidone K90 (binder) | CMS-Na | Magnesium stearate | Film coating premix (gastric-soluble) |
|---|---|---|---|---|---|---|
| Mass percentage | 64.5 | 4.5 | 1.3 | 26.4 | 0.4 | 2.9 |

### 2.2 Tablet preparation process:

### 1) Wet granulation

Povidone K90 and purified water were weighed according to the formulation.

Binder preparation: The weighed povidone K90 (binder) was added to the weighed purified water (binder solvent), followed by stirring until the solution became clear.

Premixing: The weighed povidone K90 (intragranular) and solid dispersion were sequentially added to a wet granulator, followed by mixing for 2 minutes.

Binder addition: The prepared binder was sprayed into the wet granulator.

Granulation: Water was added for granulation.

### 2) Wet milling

Wet milling was performed using an 8-mesh sieve.

### 3) Fluid bed drying

After preheating the fluid bed, the wet-milled material was fed into the fluid bed. The inlet air temperature was set to 60°C (55 to 65°C) and loss on drying was monitored. The drying process was terminated when the loss on drying was 3.0% or less.

### 4) Dry milling

Dry milling was performed using an 12-mesh sieve.

### 5) Final blending

Calculation and weighing of external excipients: The required amounts of CMS-Na and magnesium stearate were calculated according to the formulation and weighed accordingly.

Final blending: The dry-milled granules, CMS-Na, and magnesium stearate were sequentially added to a mixing hopper. The mixing speed was set to 14 rpm, followed by mixing for 4 minutes. Samples were taken to test blend uniformity.

### 6) Tableting

The samples were compressed into 150 mg tablets using a 19.0 mm × 7.6 mm oval punch.

### 7) Coating

Compound 1 (150 mg) was subjected to film coating until a coating weight gain of 3% was achieved.

### 2.3 In vivo PK study in Beagle dogs

### Experimental methods:

1) Three male Beagle dogs, conventional grade, adult males, weighing 10 to 14 kg.
2) Mode of administration: The dogs were fasted overnight, followed by intramuscular injection of 6 µg/kg pentagastrin (0.25 mg/mL), and dosing was performed 20 to 30 minutes later.
3) Dose: 300 mg/dog, approximately 30 mg/kg.
4) Route of administration: single oral dose.
5) Blood sampling points (plasma): 0, 0.25 h, 0.5 h, 1 h, 2 h, 4 h, 6 h, 8 h, 10 h, 24 h, 48 h, and 72 h (The blood sampling points for the first *in vivo* PK study were 0 to 48 h; the blood sampling points for the second *in vivo* PK study were 0 to 72 h, adding the 72 h sampling point compared to the first study).
6) Sample collection: 1 mL of blood was collected into an anticoagulant tube containing EDTA-2K, which was gently inverted to ensure thorough mixing of the anticoagulant (EDTA-2K) with the blood, then centrifuged, and rapidly stored in an ultra-low temperature freezer until sample analysis.
7) Sample analysis: Compound 1 was extracted from plasma by protein precipitation, followed by quantitative analysis using a triple quadrupole tandem mass spectrometer in multiple reaction monitoring mode. Components in the sample were separated using column chromatography and gradient elution; mobile phase: methanol and 0.1% formic acid in water; flow rate: 0.6000 mL/min; quantification range: 1.00 to 2500 ng/mL. The concentration of Compound 1 in the biological samples obtained in this experiment was determined by LC-MS/MS. Corresponding PK parameters were obtained to evaluate the PK characteristics of Compound 1 in Beagle dogs.
8) Calculation of PK parameters: The PK parameters were calculated using a non-compartmental model in Phoenix WinNonlin 8.3. The number of decimal places used in all calculations and analyses was consistent with the software output. Deviations were calculated using EXCEL software, and errors introduced by rounding did not affect data authenticity.

***In vivo* PK results (first study) (parallel comparison with Examples 3 and 5):**

| Parameter | t_{1/2} | Tₘₐₓ | Cₘₐₓ | MRT_{inf} | Cl/F | Vz/F | AUC_{0-48 h} | AUC_{0-inf} |
|---|---|---|---|---|---|---|---|---|
| Unit | h | h | ng/mL | h | mL/min/kg | L/kg | h*ng/mL | h*ng/mL |
| Mean | 19.1 | 1 | 807 | 18.7 | 79.0 | 104 | 5636 | 6462 |
| Standard deviation | 8.55 | / | 363 | 7.87 | 54.1 | 16.2 | 2639 | 3370 |

***In vivo* PK results (second study) (parallel comparison with Examples 5 and 30):**

| Parameter | t_{1/2} | Tₘₐₓ | Cₘₐₓ | MRT_{inf} | Cl/F | Vz/F | AUC_{0-48 h} | AUC_{0-inf} |
|---|---|---|---|---|---|---|---|---|
| Unit | h | h | ng/mL | h | mL/min/kg | L/kg | h*ng/mL | h*ng/mL |
| Mean | 11.1 | 1 | 673 | 12.5 | 129 | 106 | 3346 | 3445 |
| Standard deviation | 5.78 | / | 327 | 10.2 | 55.9 | 27.8 | 913 | 914 |

### 2.4 In vitro dissolution testing:

### 2.4.1 Dissolution Method 1

| **Dissolution Method 1** | Paddle method + wire sinker |
|---|---|
| Medium | pH 4.5 acetate buffer + 0.15% SDS |
| Volume | 900 mL |
| Temperature | 37°C ± 0.5°C |
| Speed | 75 rpm |
| Sampling/replenishment volume | 1.5 mL/1.5 mL |

### Dissolution results of Method 1:

| | | | | | | | | | | |
|---|---|---|---|---|---|---|---|---|---|---|
| Sampling time (min) | 5 | 10 | 15 | 20 | 30 | 45 | 60 | 75 | 90 | 100 |
| Dissolution rate (%) | 6 | 16 | 25 | 32 | 40 | 46 | 50 | 53 | 55 | 57 |

### 2.4.1 Dissolution Method 2

| **Dissolution Method 2** (two-stage dissolution) | | Paddle method + wire sinker |
|---|---|---|
| Stage 1 | Medium | Simulated gastric fluid |
| | Volume | 750 mL |
| | Temperature | 37°C ± 0.5°C |
| | Speed | 100 rpm |
| | Sampling/replenishment volume | 1.5 mL/1.5 mL |
| Stage 2 | Medium added to Stage 1 | Disodium hydrogen phosphate buffer, adjusted to pH 6.8 |
| | Temperature | 37°C ± 0.5°C |
| | Speed | 100 rpm |
| | Sampling/replenishment volume | 1.5 mL/1.5 mL |

### Dissolution results of Method 2:

| Example 2 | Simulated gastric fluid pH 1.6 | | | | | Simulated intestinal fluid pH 6.8 | | | | |
|---|---|---|---|---|---|---|---|---|---|---|
| Sampling time (min) | 5 | 15 | 30 | 45 | 60 | 5 | 15 | 30 | 60 | 75 |
| Dissolution rate (%) | 21 | 51 | 66 | 73 | 79 | 27 | 43 | 50 | 59 | 61 |

### Example 3: Dry suspension

### 3.1 Dry suspension formulation:

### Tablet Formulation 1

| Component | Solid dispersion | PVP-VA64 | Colloidal silicon dioxide | Mannitol | CMC-Na | Tartaric acid |
|---|---|---|---|---|---|---|
| Mass percentage | 50.00 | 17.50 | 4.00 | 19.25 | 3.00 | 6.25 |

### Tablet Formulation 2

| Component | Solid dispersion | PVP-VA64 | Colloidal silicon dioxide | Mannitol | CMC-Na | Tartaric acid |
|---|---|---|---|---|---|---|
| Mass percentage | 50.00 | 7.50 | 4.00 | 29.25 | 3.00 | 6.25 |

### 3.2 Preparation process:

### 1) Blending

The solid dispersion, copovidone VA64, and colloidal silicon dioxide were weighed according to the formulation and added to a mixing hopper. The mixing speed was set to 15 rpm, followed by mixing for 10 minutes. The mixture was sieved through a 40-mesh sieve. Mannitol, tartaric acid, and CMC-Na were then weighed according to the formulation and added to the mixing hopper. The mixing speed was set to 15 rpm, followed by mixing for 10 minutes. The mixture was sieved through a 40-mesh sieve.

### 3.3 In vivo PK study in Beagle dogs: The method was the same as described in Section 2.3 of Example 2, and was conducted in parallel with Example 2 and Example 5

***In vivo* PK results (first study) for Tablet Formulation 2:**

| Parameter | t_{1/2} | Tₘₐₓ | Cₘₐₓ | MRT_{inf} | Cl/F | Vz/F | AUC₀₋₄₈ h | AUC_{0-inf} |
|---|---|---|---|---|---|---|---|---|
| Unit | h | h | ng/mL | h | mL/min/kg | L/kg | h*ng/mL | h*ng/mL |
| Mean | 7.73 | 1 | 1386 | 6.35 | 61.7 | 41.4 | 7458 | 7513 |
| Standard deviation | 1.56 | / | 341 | 0.813 | 36.0 | 24.2 | 3386 | 3407 |

### Example 4: Dry suspension

### 4.1 Dry suspension formulation:

| Component | Solid dispersion | Soluplus | Colloidal silicon dioxide | Mannitol | CMC-Na | Tartaric acid |
|---|---|---|---|---|---|---|
| Mass percentage | 50.00 | 17.50 | 4.00 | 19.25 | 3.00 | 6.25 |

### 4.2 Preparation process:

### Blending

The solid dispersion, Soluplus, and colloidal silicon dioxide were weighed according to the formulation and added to a mixing hopper. The mixing speed was set to 15 rpm, followed by mixing for 10 minutes. The mixture was sieved through a 40-mesh sieve. Mannitol, tartaric acid, and CMC-Na were then weighed according to the formulation and added to the mixing hopper. The mixing speed was set to 15 rpm, followed by mixing for 10 minutes. The mixture was sieved through a 40-mesh sieve.

### Example 5: Tablet (5% tartaric acid)

### 5.1 Tablet formulation:

| Component | Solid dispersion | PVP-VA64 | Colloidal silicon dioxide | Mannitol | CCMC-Na | CMS-Na | Tartaric acid | Magnesium stearate |
|---|---|---|---|---|---|---|---|---|
| Mass percentage | 40.00 | 6.00 | 4.00 | 34.50 | 6.00 | 4.00 | 5.00 | 0.50 |

### 5.2 Preparation process:

### 1) Premixing

The solid dispersion, polymer, and colloidal silicon dioxide were weighed according to the formulation and added to a mixing hopper. The mixing speed was set to 15 rpm, followed by mixing for 10 minutes. The mixture was sieved through a 40-mesh sieve. Mannitol, organic acid, and CCMC-Na were then weighed according to the formulation and added to the mixing hopper. The mixing speed was set to 15 rpm, followed by mixing for 10 minutes. The mixture was sieved through a 40-mesh sieve.

### 2) Dry granulation

Dry granulation was performed using a dry granulator.

### 3) Dry milling

Dry milling was performed using an 12-mesh sieve.

### 4) Final blending

Calculation and weighing of external excipients: The required amounts of CMS-Na and magnesium stearate were calculated according to the formulation and weighed accordingly.

Final blending: The granules after dry granulation, CMS-Na, and magnesium stearate were added to a mixing hopper. The mixing speed was set to 15 rpm, followed by mixing for 5 minutes. Samples were taken to test blend uniformity.

### 5) Tableting

The samples were compressed into 75 mg tablets using a 16.31 mm × 8.59 mm oval punch.

### 5.3 In vivo PK study in Beagle dogs: The method was the same as described in Section 2.3 of Example 2

***In vivo* PK results (first study) (parallel comparison with Examples 2 and 3):**

| Parameter | t_{1/2} | Tₘₐₓ | Cₘₐₓ | MRT_{inf} | Cl/F | Vz/F | AUC₀₋₄₈ h | AUC_{0-inf} |
|---|---|---|---|---|---|---|---|---|
| Unit | h | h | ng/mL | h | mL/min/kg | L/kg | h*ng/mL | h*ng/mL |
| Mean | 10.5 | 1 | 1108 | 14.4 | 43.4 | 35.6 | 10470 | 11010 |
| Standard deviation | 5.67 | / | 244 | 8.06 | 18.7 | 14.4 | 4199 | 4260 |

***In vivo* PK results (second study) (parallel comparison with Examples 2 and 30):**

| Parameter | t_{1/2} | Tₘₐₓ | Cₘₐₓ | MRT_{inf} | Cl/F | Vz/F | AUC₀₋₄₈ h | AUC_{0-inf} |
|---|---|---|---|---|---|---|---|---|
| Unit | h | h | ng/mL | h | mL/min/kg | L/kg | h*ng/mL | h*ng/mL |
| Mean | 14.2 | 1 | 1750 | 20.7 | 30.8 | 37.7 | 12991 | 13938 |
| Standard deviation | 8.92 | NA | 557 | 13.2 | 13.7 | 22.5 | 6311 | 5990 |

### 5.4 In vitro dissolution testing: The method was the same as Dissolution Method 1 and Dissolution Method 2 described in Section 2.4 of Example 2

### Dissolution results of Method 1:

| | | | | | | | | | | |
|---|---|---|---|---|---|---|---|---|---|---|
| Sampling time (min) | 5 | 10 | 15 | 20 | 30 | 45 | 60 | 75 | 90 | 100 |
| Dissolution rate (%) | 26 | 37 | 45 | 51 | 59 | 68 | 73 | 77 | 78 | 83 |

### Dissolution results of Method 2:

| | Simulated gastric fluid pH 1.6 | | | | | Simulated intestinal fluid pH 6.8 | | | | |
|---|---|---|---|---|---|---|---|---|---|---|
| Sampling time (min) | 5 | 15 | 30 | 45 | 60 | 65 | 75 | 90 | 120 | 135 |
| Dissolution rate (%) | 53 | 74 | 82 | 85 | 87 | 71 | 73 | 74 | 69 | 74 |

### Example 6: Tablet (0% tartaric acid)

### 6.1 Tablet formulation:

| Component | Solid dispersion | PVP-VA64 | Colloidal silicon dioxide | Mannitol | CCMC-Na | CMS-Na | Tartaric acid | Magnesium stearate |
|---|---|---|---|---|---|---|---|---|
| Mass percentage | 40.00 | 6.00 | 4.00 | 39.50 | 6.00 | 4.00 | 0.00 | 0.50 |

### 6.2 Preparation process: Refer to Section 5.2 of Example 5

### 6.3 In vitro dissolution testing: The method was the same as Dissolution Method 1 and Dissolution Method 2 described in Section 2.4 of Example 2

### Dissolution results of Method 1:

| | | | | | | | | | | |
|---|---|---|---|---|---|---|---|---|---|---|
| Sampling time (min) | 5 | 10 | 15 | 20 | 30 | 45 | 60 | 75 | 90 | 100 |
| Dissolution rate (%) | 28 | 40 | 56 | 54 | 60 | 66 | 69 | 71 | 72 | 74 |

### Dissolution results of Method 2:

| | Simulated gastric fluid pH 1.6 | | | | | Simulated intestinal fluid pH 6.8 | | | | |
|---|---|---|---|---|---|---|---|---|---|---|
| Sampling time (min) | 5 | 15 | 30 | 45 | 60 | 65 | 75 | 90 | 120 | 135 |
| Dissolution rate (%) | 57 | 73 | 81 | 84 | 88 | 49 | 57 | 61 | 62 | 66 |

### Example 7: Tablet (10% tartaric acid)

| **7.1 Tablet formulation:**Component | Solid dispersion | PVP-VA64 | Colloidal silicon dioxide | Mannitol | CCMC-Na | CMS-Na | Tartaric acid | Magnesium stearate |
|---|---|---|---|---|---|---|---|---|
| Mass percentage | 40.00 | 6.00 | 4.00 | 29.50 | 6.00 | 4.00 | 10.00 | 0.50 |

### 7.2 Preparation process: Refer to Section 5.2 of Example 5

### 7.3 In vitro dissolution testing: The method was the same as Dissolution Method 1 and Dissolution Method 2 described in Section 2.4 of Example 2

### Dissolution results of Method 1:

| | | | | | | | | | | |
|---|---|---|---|---|---|---|---|---|---|---|
| Sampling time (min) | 5 | 10 | 15 | 20 | 30 | 45 | 60 | 75 | 90 | 100 |
| Dissolution rate (%) | 15 | 22 | 28 | 33 | 41 | 50 | 58 | 63 | 67 | 78 |

### Dissolution results of Method 2:

| | Simulated gastric fluid pH 1.6 | | | | | Simulated intestinal fluid pH 6.8 | | | | |
|---|---|---|---|---|---|---|---|---|---|---|
| Sampling time (min) | 5 | 15 | 30 | 45 | 60 | 65 | 75 | 90 | 120 | 135 |
| Dissolution rate (%) | 50 | 76 | 86 | 89 | 91 | 58 | 67 | 69 | 68 | 72 |

### Example 8: Tablet (20% tartaric acid)

### 8.1 Tablet formulation:

| Component | Solid dispersion | PVP-VA64 | Colloidal silicon dioxide | Mannitol | CCMC-Na | CMS-Na | Tartaric acid | Magnesium stearate |
|---|---|---|---|---|---|---|---|---|
| Mass percentage | 40.00 | 6.00 | 4.00 | 19.50 | 6.00 | 4.00 | 20.00 | 0.50 |

### 8.2 Preparation process: Refer to Section 5.2 of Example 5

### Example 9: Tablet (40% tartaric acid)

| **9.1 Tablet formulation:**Component | Solid dispersion | PVP-VA64 | Colloidal silicon dioxide | Mannitol | CCMC-Na | CMS-Na | Tartaric acid | Magnesium stearate |
|---|---|---|---|---|---|---|---|---|
| Mass percentage | 40.00 | 6.00 | 4.00 | 0.00 | 6.00 | 4.00 | 39.50 | 0.50 |

### 9.2 Preparation process: Refer to Section 5.2 of Example 5

### Example 10: Tablet (5% fumaric acid)

### 10.1 Tablet formulation:

| Component | Solid dispersion | PVP-VA64 | Colloidal silicon dioxide | Mannitol | CCMC-Na | CMS-Na | Fumaric acid | Magnesium stearate |
|---|---|---|---|---|---|---|---|---|
| Mass | 40.00 | 6.00 | 4.00 | 34.50 | 6.00 | 4.00 | 5.00 | 0.50 |
| percentage | | | | | | | | |

### 10.2 Preparation process: Refer to Section 5.2 of Example 5

### 10.3 In vitro dissolution testing: The method was the same as Dissolution Method 1 and Dissolution Method 2 described in Section 2.4 of Example 2

### Dissolution results of Method 1:

| | | | | | | | | | | |
|---|---|---|---|---|---|---|---|---|---|---|
| Sampling time (min) | 5 | 10 | 15 | 20 | 30 | 45 | 60 | 75 | 90 | 100 |
| Dissolution rate (%) | 24 | 42 | 55 | 64 | 75 | 84 | 88 | 90 | 91 | 92 |

### Dissolution results of Method 2:

| | Simulated gastric fluid pH 1.6 | | | | | Simulated intestinal fluid pH 6.8 | | | | |
|---|---|---|---|---|---|---|---|---|---|---|
| Sampling time (min) | 5 | 15 | 30 | 45 | 60 | 65 | 75 | 90 | 120 | 135 |
| Dissolution rate (%) | 48 | 69 | 74 | 75 | 77 | 50 | 56 | 58 | 56 | 61 |

### Example 11: Tablet (5% citric acid)

### 11.1 Tablet formulation:

| Component | Solid dispersion | PVP-VA64 | Colloidal silicon dioxide | Mannitol | CCMC-Na | CMS-Na | Citric acid | Magnesium stearate |
|---|---|---|---|---|---|---|---|---|
| Mass percentage | 40.00 | 6.00 | 4.00 | 34.50 | 6.00 | 4.00 | 5.00 | 0.50 |

### 11.2 Preparation process: Refer to Section 5.2 of Example 5

### 11.3 In vitro dissolution testing: The method was the same as Dissolution Method 1 and Dissolution Method 2 described in Section 2.4 of Example 2

### Dissolution results of Method 1:

| | | | | | | | | | | |
|---|---|---|---|---|---|---|---|---|---|---|
| Sampling time (min) | 5 | 10 | 15 | 20 | 30 | 45 | 60 | 75 | 90 | 100 |
| Dissolution rate (%) | 20 | 33 | 44 | 53 | 66 | 78 | 83 | 87 | 90 | 92 |

### Dissolution results of Method 2:

| | Simulated gastric fluid pH 1.6 | | | | | Simulated intestinal fluid pH 6.8 | | | | |
|---|---|---|---|---|---|---|---|---|---|---|
| Sampling time (min) | 5 | 15 | 30 | 45 | 60 | 65 | 75 | 90 | 120 | 135 |
| Dissolution rate (%) | 64 | 84 | 90 | 92 | 93 | 52 | 63 | 66 | 65 | 69 |

### Example 12: Tablet (5% malic acid)

### 12.1 Tablet formulation:

| Component | Solid dispersion | PVP-VA64 | Colloidal silicon dioxide | Mannitol | CCMC-Na | CMS-Na | Malic acid | Magnesium stearate |
|---|---|---|---|---|---|---|---|---|
| Mass percentage | 40.00 | 6.00 | 4.00 | 34.50 | 6.00 | 4.00 | 5.00 | 0.50 |

### 12.2 Preparation process: Refer to Section 5.2 of Example 5

### 12.3 In vitro dissolution testing: The method was the same as Dissolution Method 1 and Dissolution Method 2 described in Section 2.4 of Example 2

### Dissolution results of Method 1:

| | | | | | | | | | | |
|---|---|---|---|---|---|---|---|---|---|---|
| Sampling time (min) | 5 | 10 | 15 | 20 | 30 | 45 | 60 | 75 | 90 | 100 |
| Dissolution rate (%) | 20 | 31 | 39 | 46 | 55 | 63 | 69 | 73 | 76 | 83 |

### Dissolution results of Method 2:

| | Simulated gastric fluid pH 1.6 | | | | | Simulated intestinal fluid pH 6.8 | | | | |
|---|---|---|---|---|---|---|---|---|---|---|
| Sampling time (min) | 5 | 15 | 30 | 45 | 60 | 65 | 75 | 90 | 120 | 135 |
| Dissolution rate (%) | 37 | 65 | 79 | 84 | 89 | 51 | 62 | 63 | 66 | 71 |

### Example 13: Tablet (6% Soluplus)

### 13.1 Tablet formulation:

| Component | Solid dispersion | Soluplus | Colloidal silicon dioxide | Mannitol | CCMC-Na | CMS-Na | Tartaric acid | Magnesium stearate |
|---|---|---|---|---|---|---|---|---|
| Mass percentage | 40.00 | 6.00 | 4.00 | 34.50 | 6.00 | 4.00 | 5.00 | 0.50 |

### 13.2 Preparation process: Refer to Section 5.2 of Example 5

### 13.3 In vitro dissolution testing: The method was the same as Dissolution Method 1 and Dissolution Method 2 described in Section 2.4 of Example 2

### Dissolution results of Method 1:

| | | | | | | | | | | |
|---|---|---|---|---|---|---|---|---|---|---|
| Sampling time (min) | 5 | 10 | 15 | 20 | 30 | 45 | 60 | 75 | 90 | 100 |
| Dissolution rate (%) | 22 | 35 | 46 | 54 | 65 | 75 | 81 | 84 | 87 | 92 |

### Dissolution results of Method 2:

| | Simulated gastric fluid pH 1.6 | | | | | Simulated intestinal fluid pH 6.8 | | | | |
|---|---|---|---|---|---|---|---|---|---|---|
| Sampling time (min) | 5 | 15 | 30 | 45 | 60 | 65 | 75 | 90 | 120 | 135 |
| Dissolution rate (%) | 50 | 71 | 79 | 82 | 88 | 53 | 62 | 68 | 72 | 75 |

### Example 14: Tablet (6% HPMC E3LV)

### 14.1 Tablet formulation:

| Component | Solid dispersion | **HPMC E3LV** | Colloidal silicon dioxide | Mannitol | CCMC-Na | CMS-Na | Tartaric acid | Magnesium stearate |
|---|---|---|---|---|---|---|---|---|
| Mass percentage | 40.00 | 6.00 | 4.00 | 34.50 | 6.00 | 4.00 | 5.00 | 0.50 |

### 14.2 Preparation process: Refer to Section 5.2 of Example 5

### 14.3 In vitro dissolution testing: The method was the same as Dissolution Method 1 and Dissolution Method 2 described in Section 2.4 of Example 2

### Dissolution results of Method 1:

| | | | | | | | | | | |
|---|---|---|---|---|---|---|---|---|---|---|
| Sampling time (min) | 5 | 10 | 15 | 20 | 30 | 45 | 60 | 75 | 90 | 100 |
| Dissolution rate (%) | 19 | 31 | 40 | 48 | 60 | 72 | 78 | 83 | 85 | 90 |

### Dissolution results of Method 2:

| | Simulated gastric fluid pH 1.6 | | | | | Simulated intestinal fluid pH 6.8 | | | | |
|---|---|---|---|---|---|---|---|---|---|---|
| Sampling time (min) | 5 | 15 | 30 | 45 | 60 | 65 | 75 | 90 | 120 | 135 |
| Dissolution rate (%) | 43 | 67 | 78 | 82 | 86 | 41 | 47 | 52 | 55 | 61 |

### Example 15: Tablet (6% PVP K30)

### 15.1 Tablet formulation:

| Component | Solid dispersion | **PVP K30** | Colloidal silicon dioxide | Mannitol | CCMC-Na | CMS-Na | Tartaric acid | Magnesium stearate |
|---|---|---|---|---|---|---|---|---|
| Mass percentage | 40.00 | 6.00 | 4.00 | 34.50 | 6.00 | 4.00 | 5.00 | 0.50 |

### 15.2 Preparation process: Refer to Section 5.2 of Example 5

### 15.3 In vitro dissolution testing: The method was the same as Dissolution Method 1 and Dissolution Method 2 described in Section 2.4 of Example 2

### Dissolution results of Method 1:

| | | | | | | | | | | |
|---|---|---|---|---|---|---|---|---|---|---|
| Sampling time (min) | 5 | 10 | 15 | 20 | 30 | 45 | 60 | 75 | 90 | 100 |
| Dissolution rate (%) | 19 | 31 | 39 | 46 | 54 | 64 | 69 | 73 | 76 | 85 |

### Dissolution results of Method 2:

| | Simulated gastric fluid pH 1.6 | | | | | Simulated intestinal fluid pH 6.8 | | | | |
|---|---|---|---|---|---|---|---|---|---|---|
| Sampling time (min) | 5 | 15 | 30 | 45 | 60 | 65 | 75 | 90 | 120 | 135 |
| Dissolution rate (%) | 55 | 73 | 80 | 83 | 86 | 44 | 50 | 67 | 69 | 68 |

### Example 16: Tablet (0% PVP VA64)

### 16.1 Tablet formulation:

| Component | Solid dispersion | PVP VA 64 | Colloidal silicon dioxide | Mannitol | CCMC-Na | CMS-Na | Citric acid | Magnesium stearate |
|---|---|---|---|---|---|---|---|---|
| Mass percentage | 40.00 | 0 | 4.00 | 40.50 | 6.00 | 4.00 | 5.00 | 0.50 |

### 16.2 Preparation process: Refer to Section 5.2 of Example 5

### 16.3 In vitro dissolution testing: The method was the same as Dissolution Method 1 and Dissolution Method 2 described in Section 2.4 of Example 2

### Dissolution results of Method 1:

| | | | | | | | | | | |
|---|---|---|---|---|---|---|---|---|---|---|
| Sampling time (min) | 5 | 10 | 15 | 20 | 30 | 45 | 60 | 75 | 90 | 100 |
| Dissolution rate (%) | 22 | 36 | 47 | 55 | 65 | 74 | 79 | 82 | 84 | 89 |

### Dissolution results of Method 2:

| | Simulated gastric fluid pH 1.6 | | | | | Simulated intestinal fluid pH 6.8 | | | | |
|---|---|---|---|---|---|---|---|---|---|---|
| Sampling time (min) | 5 | 15 | 30 | 45 | 60 | 65 | 75 | 90 | 120 | 135 |
| Dissolution rate (%) | 44 | 75 | 85 | 89 | 94 | 45 | 62 | 68 | 72 | 76 |

### Example 17: Tablet (12% PVP VA64)

### 17.1 Tablet formulation:

| Component | Solid dispersion | PVP VA 64 | Colloidal silicon dioxide | Mannitol | CCMC-Na | CMS-Na | Citric acid | Magnesium stearate |
|---|---|---|---|---|---|---|---|---|
| Mass percentage | 40.00 | 12.00 | 4.00 | 28.5 | 6.00 | 4.00 | 5.00 | 0.50 |

### 17.2 Preparation process: Refer to Section 5.2 of Example 5

### 17.3 In vitro dissolution testing: The method was the same as Dissolution Method 1 and Dissolution Method 2 described in Section 2.4 of Example 2

### Dissolution results of Method 1:

| | | | | | | | | | | |
|---|---|---|---|---|---|---|---|---|---|---|
| Sampling time (min) | 5 | 10 | 15 | 20 | 30 | 45 | 60 | 75 | 90 | 100 |
| Dissolution rate (%) | 26 | 39 | 47 | 54 | 64 | 73 | 79 | 83 | 85 | 89 |

### Dissolution results of Method 2:

| | Simulated gastric fluid pH 1.6 | | | | | Simulated intestinal fluid pH 6.8 | | | | |
|---|---|---|---|---|---|---|---|---|---|---|
| Sampling time (min) | 5 | 15 | 30 | 45 | 60 | 65 | 75 | 90 | 120 | 135 |
| Dissolution rate (%) | 73 | 87 | 91 | 93 | 94 | 40 | 61 | 67 | 73 | 78 |

### Example 18: Tablet (24% PVP VA64)

### 18.1 Tablet formulation:

| Component | Solid dispersion | PVP VA 64 | Colloidal silicon dioxide | Mannitol | CCMC-Na | CMS-Na | Citric acid | Magnesium stearate |
|---|---|---|---|---|---|---|---|---|
| Mass percentage | 40.00 | 24.00 | 4.00 | 16.5 | 6.00 | 4.00 | 5.00 | 0.50 |

### 18.2 Preparation process: Refer to Section 5.2 of Example 5

### 18.3 In vitro dissolution testing: The method was the same as Dissolution Method 1 and Dissolution Method 2 described in Section 2.4 of Example 2

### Dissolution results of Method 1:

| | | | | | | | | | | |
|---|---|---|---|---|---|---|---|---|---|---|
| Sampling time (min) | 5 | 10 | 15 | 20 | 30 | 45 | 60 | 75 | 90 | 100 |
| Dissolution rate (%) | 22 | 35 | 44 | 52 | 64 | 76 | 82 | 86 | 89 | 91 |

### Dissolution results of Method 2:

| | Simulated gastric fluid pH 1.6 | | | | | Simulated intestinal fluid pH 6.8 | | | | |
|---|---|---|---|---|---|---|---|---|---|---|
| Sampling time (min) | 5 | 15 | 30 | 45 | 60 | 65 | 75 | 90 | 120 | 135 |
| Dissolution rate (%) | 78 | 90 | 92 | 93 | 93 | 45 | 60 | 69 | 73 | 77 |

### Example 19: Tablet

### 19.1 Tablet formulation:

| Component | Solid dispersion | Soluplus | Colloidal silicon dioxide | Mannitol | CCMC-Na | CMS-Na | Tartaric acid | Magnesium stearate |
|---|---|---|---|---|---|---|---|---|
| Mass percentage | 40.00 | 14.00 | 4.00 | 26.50 | 6.00 | 4.00 | 5.00 | 0.50 |

### 19.2 Preparation process: Refer to Section 5.2 of Example 5

### 19.3 In vitro dissolution testing:

| **Dissolution Method 3** | Paddle method + wire sinker |
|---|---|
| Medium | Simulated gastric fluid |
| Volume | 900 mL |
| Temperature | 37°C ± 0.5°C |
| Speed | 100 rpm |
| Sampling/replenishment volume | 1.5 mL/1.5 mL |

### Dissolution results of Method 3:

| | Simulated gastric fluid pH 1.6 | | | |
|---|---|---|---|---|
| Sampling time (min) | 5 | 30 | 60 | 75 |
| Dissolution rate (%) | 64 | 82 | 85 | 87 |

### Example 20: Tablet

### 20.1 Tablet formulation:

| Component | Solid dispersion | Colloidal silicon dioxide | Mannitol | CCMC-Na | CMS-Na | Tartaric acid | SDS | Magnesium stearate |
|---|---|---|---|---|---|---|---|---|
| Mass percentage | 40.00 | 4.00 | 35.50 | 6.00 | 4.00 | 5.00 | 5.00 | 0.50 |

### 20.2 Preparation process:

### 1) Premixing

The solid dispersion, SDS, and colloidal silicon dioxide were weighed according to the formulation and added to a mixing hopper. The mixing speed was set to 15 rpm, followed by mixing for 10 minutes. The mixture was sieved through a 40-mesh sieve. Mannitol, tartaric acid, and CCMC-Na were then weighed according to the formulation and added to the mixing hopper. The mixing speed was set to 15 rpm, followed by mixing for 10 minutes. The mixture was sieved through a 40-mesh sieve.

### 2) Dry granulation

Dry granulation was performed using a dry granulator.

### 3) Dry milling

Dry milling was performed using an 12-mesh sieve.

### 4) Final blending

Calculation and weighing of external excipients: The required amounts of CMS-Na and magnesium stearate were calculated according to the formulation and weighed accordingly.

Final blending: The granules after dry granulation, CMS-Na, and magnesium stearate were added to a mixing hopper. The mixing speed was set to 15 rpm, followed by mixing for 5 minutes. Samples were taken to test blend uniformity.

### 5) Tableting

The samples were compressed into 75 mg tablets using a 16.31 mm × 8.59 mm oval punch.

### 20.3 In vitro dissolution testing:

| **Dissolution Method 3** | Paddle method + wire sinker |
|---|---|
| Medium | Simulated gastric fluid |
| Volume | 900 mL |
| Temperature | 37°C ± 0.5°C |
| Speed | 100 rpm |
| Sampling/replenishment volume | 1.5 mL/1.5 mL |

### Example 21: Tablet

### 21.1 Tablet formulation:

| Component | Solid dispersion | Soluplus | SDS | Mannitol | CCMC-Na | CMS-Na | Tartaric acid | Magnesium stearate |
|---|---|---|---|---|---|---|---|---|
| Mass percentage | 40.00 | 14.00 | 5.00 | 25.50 | 6.00 | 4.00 | 5.00 | 0.50 |

### 21.2 Preparation process:

### 1) Premixing

The solid dispersion, Soluplus, and SDS were weighed according to the formulation and added to a mixing hopper. The mixing speed was set to 15 rpm, followed by mixing for 10 minutes. The mixture was sieved through a 40-mesh sieve. Mannitol, tartaric acid, and CCMC-Na were then weighed according to the formulation and added to the mixing hopper. The mixing speed was set to 15 rpm, followed by mixing for 10 minutes. The mixture was sieved through a 40-mesh sieve.

### 2) Dry granulation

Dry granulation was performed using a dry granulator.

### 3) Dry milling

Dry milling was performed using an 12-mesh sieve.

### 4) Final blending

Calculation and weighing of external excipients: The required amounts of CMS-Na and magnesium stearate were calculated according to the formulation and weighed accordingly.

Final blending: The granules after dry granulation, CMS-Na, and magnesium stearate were added to a mixing hopper. The mixing speed was set to 15 rpm, followed by mixing for 5 minutes. Samples were taken to test blend uniformity.

### 5) Tableting

The samples were compressed into 75 mg tablets using a 16.31 mm × 8.59 mm oval punch.

### 21.3 In vitro dissolution testing:

| **Dissolution Method 3** | Paddle method + wire sinker |
|---|---|
| Medium | Simulated gastric fluid |
| Volume | 900 mL |
| Temperature | 37°C ± 0.5°C |
| Speed | 100 rpm |
| Sampling/replenishment volume | 1.5 mL/1.5 mL |

### Example 22: Tablet

### 22.1 Tablet formulation:

| Component | Solid dispersion | Soluplus | Colloidal silicon dioxide | Mannitol | CCMC-Na | CMS-Na | SDS | Magnesium stearate |
|---|---|---|---|---|---|---|---|---|
| Mass percentage | 40.00 | 14.00 | 4.00 | 26.50 | 6.00 | 4.00 | 5.00 | 0.50 |

### 22.2 Preparation process:

### 1) Premixing

The solid dispersion, Soluplus, colloidal silicon dioxide, and SDS were weighed according to the formulation and added to a mixing hopper. The mixing speed was set to 15 rpm, followed by mixing for 10 minutes. The mixture was sieved through a 40-mesh sieve. Mannitol and CCMC-Na were then weighed according to the formulation and added to the mixing hopper. The mixing speed was set to 15 rpm, followed by mixing for 10 minutes. The mixture was sieved through a 40-mesh sieve.

### 2) Dry granulation

Dry granulation was performed using a dry granulator.

### 3) Dry milling

Dry milling was performed using an 12-mesh sieve.

### 4) Final blending

Calculation and weighing of external excipients: The required amounts of CMS-Na and magnesium stearate were calculated according to the formulation and weighed accordingly.

Final blending: The granules after dry granulation, CMS-Na, and magnesium stearate were added to a mixing hopper. The mixing speed was set to 15 rpm, followed by mixing for 5 minutes. Samples were taken to test blend uniformity.

### 5) Tableting

The samples were compressed into 75 mg tablets using a 16.31 mm × 8.59 mm oval punch.

### 22.3 In vitro dissolution testing:

| **Dissolution Method 3** | Paddle method + wire sinker |
|---|---|
| Medium | Simulated gastric fluid |
| Volume | 900 mL |
| Temperature | 37°C ± 0.5°C |
| Speed | 100 rpm |
| Sampling/replenishment volume | 1.5 mL/1.5 mL |

### Example 23: Tablet

### 23.1 Tablet formulation:

| Component | Solid dispersion | PVP K30 | CMS-Na | Magnesium stearate |
|---|---|---|---|---|
| Mass percentage | 66.40 | 6.00 | 27.20 | 0.40 |

### 23.2 Preparation process:

### 1) Premixing

The solid dispersion and PVP K30 were weighed according to the formulation and added to a mixing hopper.

### 2) Dry granulation

Dry granulation was performed using a dry granulator.

### 3) Dry milling

Dry milling was performed using an 12-mesh sieve.

### 4) Final blending

Calculation and weighing of external excipients: The required amounts of CMS-Na and magnesium stearate were calculated according to the formulation and weighed accordingly.

Final blending: The granules after dry granulation, CMS-Na, and magnesium stearate were added to a mixing hopper. The mixing speed was set to 15 rpm, followed by mixing for 5 minutes. Samples were taken to test blend uniformity.

### 5) Tableting

The samples were compressed into 75 mg tablets using a 16.31 mm × 8.59 mm oval punch.

### 23.3 In vitro dissolution testing:

| **Dissolution Method 3** | Paddle method + wire sinker |
|---|---|
| Medium | Simulated gastric fluid |
| Volume | 900 mL |
| Temperature | 37°C ± 0.5°C |
| Speed | 100 rpm |
| Sampling/replenishment volume | 1.5 mL/1.5 mL |

### Dissolution results of Method 3:

| | Simulated gastric fluid pH 1.6 | | | |
|---|---|---|---|---|
| Sampling time (min) | 5 | 30 | 60 | 75 |
| Dissolution rate (%) | 77 | 91 | 93 | 92 |

### Example 24: Tablet

### 24.1 Tablet formulation:

| Component | Solid dispersion | Soluplus | Colloidal silicon dioxide | Mannitol | CMS-Na | Tartaric acid | Magnesium stearate |
|---|---|---|---|---|---|---|---|
| Mass percentage | 40.00 | 14.00 | 4.00 | 26.50 | 10.00 | 5.00 | 0.50 |

### 24.2 Preparation process:

### 1) Premixing

The solid dispersion, Soluplus, and colloidal silicon dioxide were weighed according to the formulation and added to a mixing hopper. The mixing speed was set to 15 rpm, followed by mixing for 10 minutes. The mixture was sieved through a 40-mesh sieve. Mannitol and tartaric acid were then weighed according to the formulation and added to the mixing hopper. The mixing speed was set to 15 rpm, followed by mixing for 10 minutes. The mixture was sieved through a 40-mesh sieve.

### 2) Dry granulation

Dry granulation was performed using a dry granulator.

### 3) Dry milling

Dry milling was performed using an 12-mesh sieve.

### 4) Final blending

Calculation and weighing of external excipients: The required amounts of CMS-Na and magnesium stearate were calculated according to the formulation and weighed accordingly.

Final blending: The granules after dry granulation, CMS-Na, and magnesium stearate were added to a mixing hopper. The mixing speed was set to 15 rpm, followed by mixing for 5 minutes. Samples were taken to test blend uniformity.

### 5) Tableting

The samples were compressed into 75 mg tablets using a 16.31 mm × 8.59 mm oval punch.

### 24.3 In vitro dissolution testing:

| **Dissolution Method 3** | Paddle method + wire sinker |
|---|---|
| Medium | Simulated gastric fluid |
| Volume | 900 mL |
| Temperature | 37°C ± 0.5°C |
| Speed | 100 rpm |
| Sampling/replenishment volume | 1.5 mL/1.5 mL |

### Dissolution results of Method 3:

| | Simulated gastric fluid pH 1.6 | | | |
|---|---|---|---|---|
| Sampling time (min) | 5 | 30 | 60 | 75 |
| Dissolution rate (%) | 24 | 43 | 56 | 82 |

### Example 25: Tablet

### 25.1 Tablet formulation:

| Component | Solid dispersion | Soluplus | Colloidal silicon dioxide | Mannitol | CCMC-Na | CMS-Na | Tartaric acid | SDS | Magnesium stearate |
|---|---|---|---|---|---|---|---|---|---|
| Mass percentage | 40.00 | 14.00 | 4.00 | 21.50 | 6.00 | 4.00 | 5.00 | 5.00 | 0.50 |

### 25.2 Preparation process:

### 1) Premixing

The solid dispersion, Soluplus, colloidal silicon dioxide, and SDS were weighed according to the formulation and added to a mixing hopper. The mixing speed was set to 15 rpm, followed by mixing for 10 minutes. The mixture was sieved through a 40-mesh sieve. Mannitol, tartaric acid, and CCMC-Na were then weighed according to the formulation and added to the mixing hopper. The mixing speed was set to 15 rpm, followed by mixing for 10 minutes. The mixture was sieved through a 40-mesh sieve.

### 2) Dry granulation

Dry granulation was performed using a dry granulator.

### 3) Dry milling

Dry milling was performed using an 12-mesh sieve.

### 4) Final blending

Calculation and weighing of external excipients: The required amounts of CMS-Na and magnesium stearate were calculated according to the formulation and weighed accordingly.

Final blending: The granules after dry granulation, CMS-Na, and magnesium stearate were added to a mixing hopper. The mixing speed was set to 15 rpm, followed by mixing for 5 minutes. Samples were taken to test blend uniformity.

### 5) Tableting

The samples were compressed into 75 mg tablets using a 16.31 mm × 8.59 mm oval punch.

### Example 26: Tablet

### 26.1 Tablet formulation:

### Tablet Formulation 1

| Component | Solid dispersion | PVP-VA64 | Colloidal silicon dioxide | Mannitol | CCMC-Na | CMS-Na | Tartaric acid | SDS | Magnesium stearate |
|---|---|---|---|---|---|---|---|---|---|
| Mass percentage | 40.00 | 14.00 | 4.00 | 21.50 | 6.00 | 4.00 | 5.00 | 5.00 | 0.50 |

### Tablet Formulation 2

| Component | Solid dispersion | PVP-VA64 | Colloidal silicon dioxide | Mannitol | CCMC-Na | CMS-Na | Tartaric acid | SDS | Magnesium stearate |
|---|---|---|---|---|---|---|---|---|---|
| Mass percentage | 40.00 | 6.00 | 4.00 | 29.50 | 6.00 | 4.00 | 5.00 | 5.00 | 0.50 |

### 26.2 Preparation process:

### 1) Premixing

The solid dispersion, copovidone VA64, colloidal silicon dioxide, and SDS were weighed according to the formulation and added to a mixing hopper. The mixing speed was set to 15 rpm, followed by mixing for 10 minutes. The mixture was sieved through a 40-mesh sieve. Mannitol, tartaric acid, and CCMC-Na were then weighed according to the formulation and added to the mixing hopper. The mixing speed was set to 15 rpm, followed by mixing for 10 minutes. The mixture was sieved through a 40-mesh sieve.

### 2) Dry granulation

Dry granulation was performed using a dry granulator.

### 3) Dry milling

Dry milling was performed using an 12-mesh sieve.

### 4) Final blending

Calculation and weighing of external excipients: The required amounts of CMS-Na and magnesium stearate were calculated according to the formulation and weighed accordingly.

Final blending: The granules after dry granulation, CMS-Na, and magnesium stearate were added to a mixing hopper. The mixing speed was set to 15 rpm, followed by mixing for 5 minutes. Samples were taken to test blend uniformity.

### 5) Tableting

The samples were compressed into 75 mg tablets using a 16.31 mm × 8.59 mm oval punch.

### Example 27: Tablet (2% CMS-Na)

### 27.1 Tablet formulation:

| Component | Solid dispersion | PVP-VA64 | Colloidal silicon dioxide | Mannitol | CCMC-Na | CMS-Na | Tartaric acid | Magnesium stearate |
|---|---|---|---|---|---|---|---|---|
| Mass percentage | 40.00 | 6.00 | 4.00 | 36.50 | 6.00 | 2.00 | 5.00 | 0.50 |

### 27.2 Preparation process: Refer to Section 5.2 of Example 5

### 27.3 In vitro dissolution testing: The method was the same as Dissolution Method 2 described in Section 2.4.2 of Example 2

### Dissolution results of Method 2:

| | Simulated gastric fluid pH 1.6 | | | | | Simulated intestinal fluid pH 6.8 | | | | |
|---|---|---|---|---|---|---|---|---|---|---|
| Sampling time (min) | 5 | 15 | 30 | 45 | 60 | 65 | 75 | 90 | 120 | 135 |
| Dissolution rate (%) | 73 | 87 | 91 | 93 | 93 | 46 | 65 | 68 | 70 | 72 |

### Example 28: Tablet (8% CMS-Na)

### 28.1 Tablet formulation:

| Component | Solid dispersion | PVP-VA64 | Colloidal silicon dioxide | Mannitol | CCMC-Na | CMS-Na | Tartaric acid | Magnesium stearate |
|---|---|---|---|---|---|---|---|---|
| Mass percentage | 40.00 | 6.00 | 4.00 | 30.50 | 6.00 | 8.00 | 5.00 | 0.50 |

### 28.2 Preparation process: Refer to Section 5.2 of Example 5

### 28.3 In vitro dissolution testing: The method was the same as Dissolution Method 2 described in Section 2.4 of Example 2

### Dissolution results of Method 2:

| | Simulated gastric fluid pH 1.6 | | | | | Simulated intestinal fluid pH 6.8 | | | | |
|---|---|---|---|---|---|---|---|---|---|---|
| Sampling time (min) | 5 | 15 | 30 | 45 | 60 | 65 | 75 | 90 | 120 | 135 |
| Dissolution rate (%) | 68 | 86 | 90 | 92 | 92 | 55 | 70 | 75 | 76 | 79 |

### Example 29: Tablet

### 29.1 Tablet formulation:

| Component | Solid dispersion | PVP-VA64 | Colloidal silicon dioxide | Mannitol | CCMC-Na | CMS-Na | Tartaric acid | Magnesium stearate |
|---|---|---|---|---|---|---|---|---|
| Mass percentage | 50.00 | 0.00 | 4.00 | 35.50 | 6.00 | 4.00 | 0.00 | 0.50 |

### 29.2 Preparation process: Refer to Section 5.2 of Example 5

### 29.3 In vitro dissolution testing: The method was the same as Dissolution Method 2 described in Section 2.4.2 of Example 2

### Dissolution results of Method 2:

| | Simulated gastric fluid pH 1.6 | | | | | Simulated intestinal fluid pH 6.8 | | | | |
|---|---|---|---|---|---|---|---|---|---|---|
| Sampling time (min) | 5 | 15 | 30 | 45 | 60 | 65 | 75 | 90 | 120 | 135 |
| Dissolution rate (%) | 42 | 53 | 59 | 62 | 80 | 31 | 46 | 51 | 56 | 62 |

### Example 30: Tablet

### 30.1 Tablet formulation:

| Component | Solid dispersion | Soluplus | Colloidal silicon dioxide | Mannitol | CCMC-Na | CMS-Na | Tartaric acid | Magnesium stearate |
|---|---|---|---|---|---|---|---|---|
| Mass percentage | 40.00 | 12.00 | 4.00 | 28.50 | 6.00 | 4.00 | 5.00 | 0.50 |

### 30.2 Preparation process: Refer to Section 5.2 of Example 5

### 30.3 In vivo PK study in Beagle dogs: The method was the same as described in Section 2.3 of Example 2

### In vivo PK results (second study) (parallel comparison with Examples 2 and 5):

| Parameter | t_{1/2} | Tₘₐₓ | Cₘₐₓ | MRT_{inf} | Cl/F | Vz/F | AUC₀₋₄₈ h | AUC_{0-inf} |
|---|---|---|---|---|---|---|---|---|
| Unit | h | h | ng/mL | h | mL/min/kg | L/kg | h*ng/mL | h*ng/mL |
| Mean | 7.96 | 1 | 1433 | 8.68 | 40.7 | 27.9 | 10678 | 10715 |
| Standard deviation | 1.46 | NA | 56.9 | 1.80 | 2.53 | 3.97 | 1644 | 1652 |

### Example 31: Screening of surfactant content

| Material | Mass percentage/% | | |
|---|---|---|---|
| | Formulation 1 | Formulation 2 | Formulation 3 |
| Solid dispersion | 40.00 | 40.00 | 40.00 |
| PVP-VA64 | 12.00 | 12.00 | 12.00 |
| Colloidal silicon dioxide | 4.00 | 4.00 | 4.00 |
| Mannitol | 22.50 | 18.50 | 13.50 |
| CCMC-Na | 6.00 | 6.00 | 6.00 |
| CMS-Na | 4.00 | 4.00 | 4.00 |
| Magnesium stearate | 0.50 | 0.50 | 0.50 |
| Tartaric acid | 10.00 | 10.00 | 10.00 |
| Poloxamer 407 | 1.00 | 5.00 | 10.00 |
| Total | 100.00 | 100.00 | 100.00 |

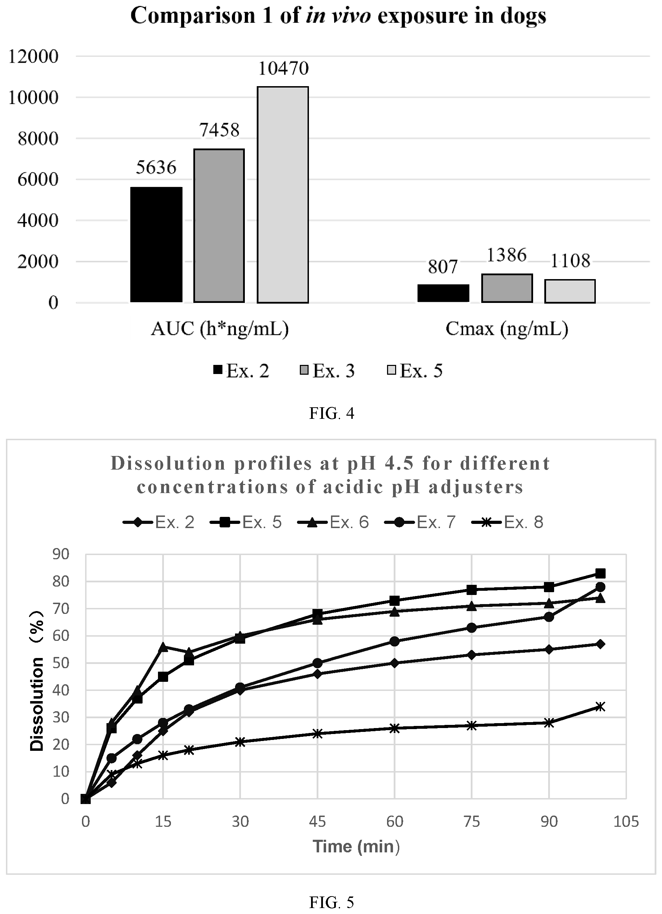

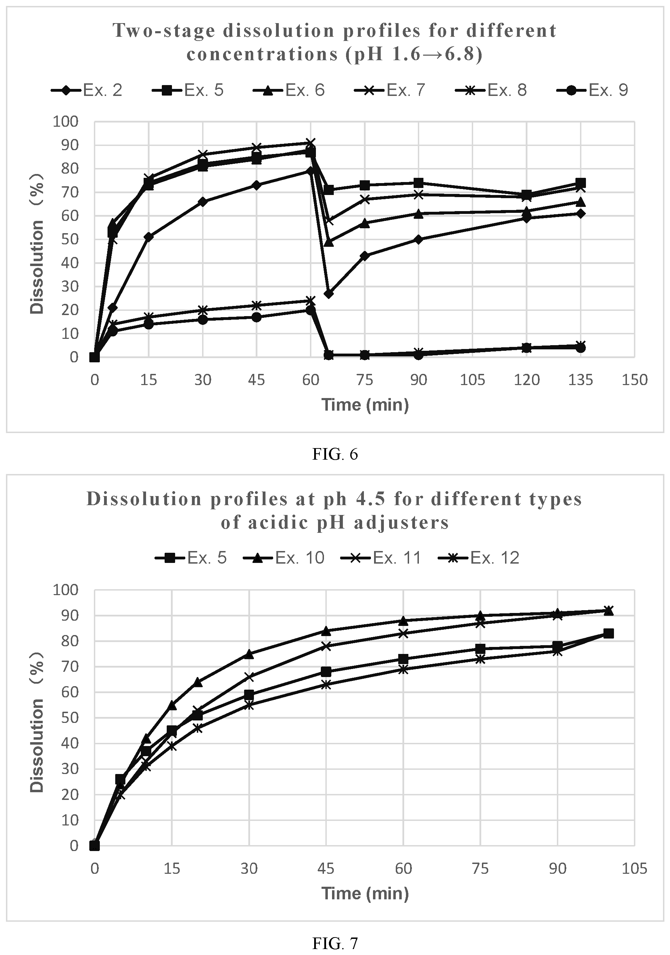

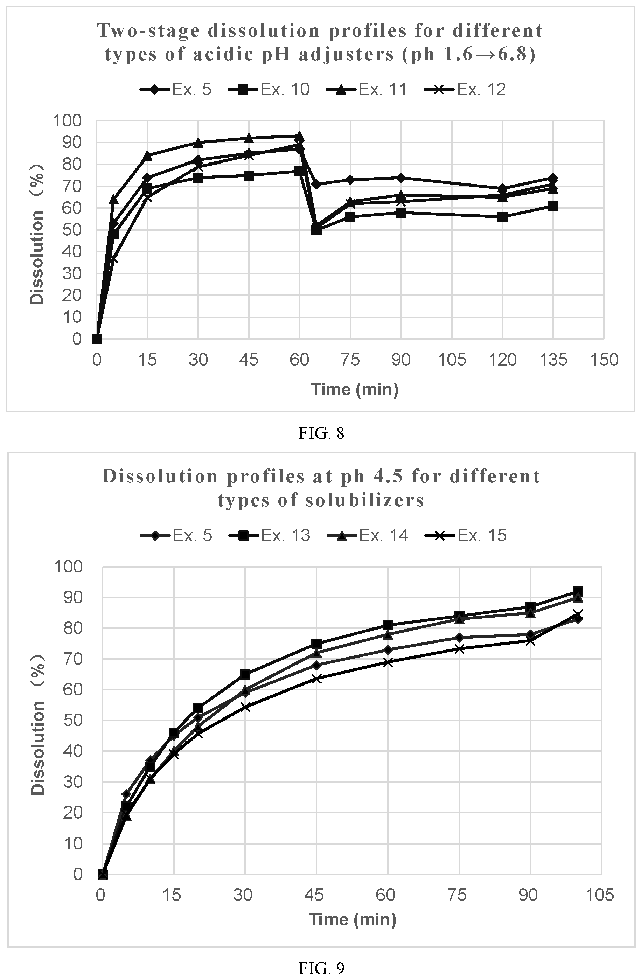

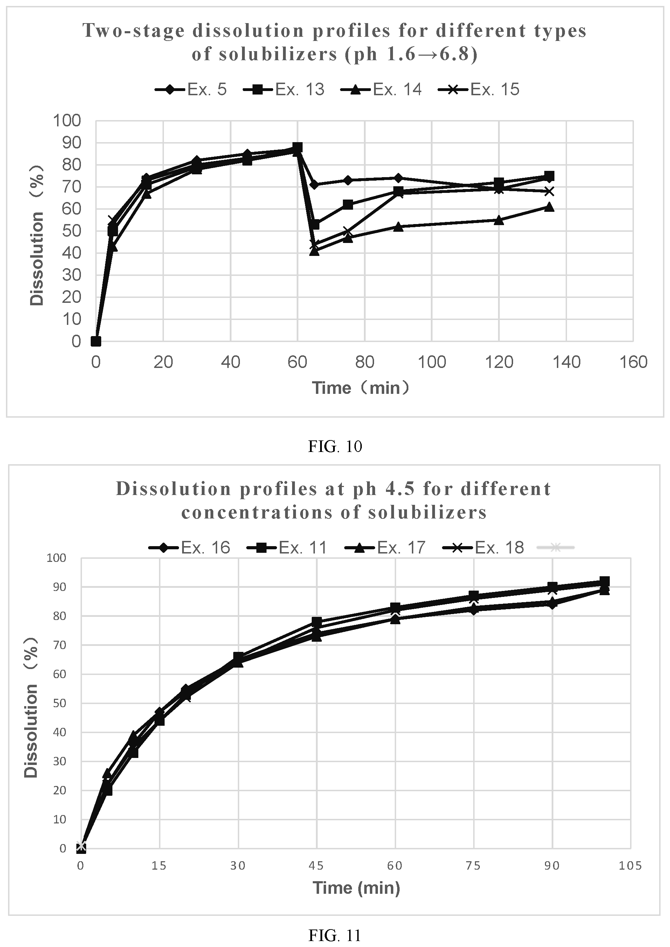

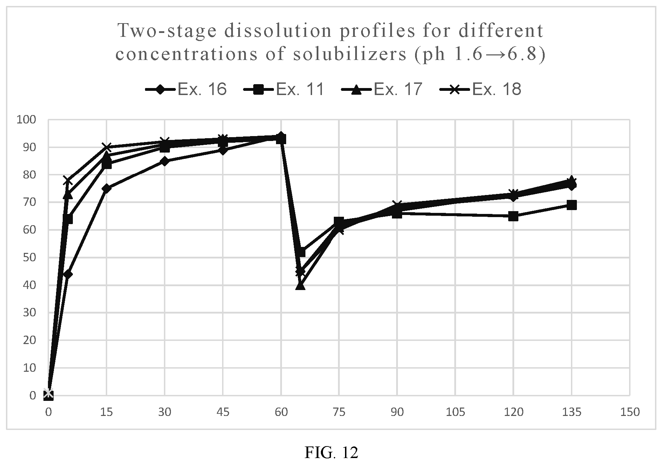

The comparative results of *in vivo* PK studies and *in vitro* dissolution testing described in the above examples are shown in FIGs. 4, 5, 6, 7, 8, 9, 10, 11, and 12.

Although specific embodiments of the present disclosure have been described above, those skilled in the art should understand that these embodiments are merely illustrative, and various changes or modifications may be made to these embodiments without departing from the principle and essence of the present disclosure. Therefore, the scope of protection of the present disclosure is defined by the appended claims.

## Claims

1. A solid dispersion, wherein the solid dispersion comprises the following components: Compound 1 and a polymeric carrier; 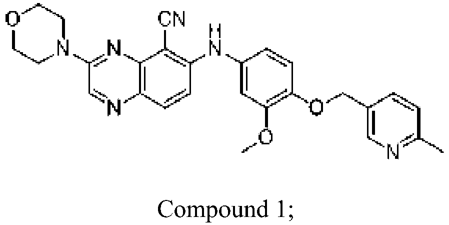 wherein the polymeric carrier is selected from one or more of HPMCAS, Soluplus, and PVP-VA.

2. The solid dispersion according to claim 1, wherein the solid dispersion satisfies one or more of the following conditions:
(1) the polymeric carrier is HPMCAS, preferably selected from one or both of HPMCAS-HF and HPMCAS-HG, more preferably HPMCAS-HG; or, the polymeric carrier is Soluplus; or, the polymeric carrier is PVP-VA, preferably PVP-VA64;
(2) in the solid dispersion, the weight ratio of Compound 1 to the polymeric carrier is approximately 1:1 to approximately 1:10; preferably approximately 1:2 to approximately 1:5, such as 3:7, 1:3, or 1:4; more preferably approximately 1:3 to approximately 1:4;
(3) in the solid dispersion, the polymeric carrier is present in an amount of at least approximately 50%, at least approximately 60%, at least approximately 67%, at least approximately 70%, at least approximately 75%, at least approximately 80%, or at least approximately 90% by weight of the solid dispersion;
(4) in the solid dispersion, Compound 1 is present in an amount of approximately 10% to approximately 50%, approximately 20% to approximately 40%, or approximately 25% to approximately 33% by weight of the solid dispersion;
(5) the solid dispersion further comprises an excipient, such as a surfactant, a glidant, or a plasticizer;
(6) in the solid dispersion, Compound 1 is dispersed in the polymeric carrier in an amorphous or near-amorphous or substantially non-crystalline state;
(7) the solid dispersion is obtained by spray drying, hot-melt extrusion, or solvent evaporation;
(8) the solid dispersion consists of Compound 1 and the polymeric carrier.

3. The solid dispersion according to claim 2, wherein the solid dispersion consists of components in mass percentages according to any one of the following schemes:
scheme 1.1: 25% Compound 1 and 75% Soluplus;
scheme 1.2: 25% Compound 1 and 75% Plasdone^{™} S-630 ULTRA;
scheme 1.3: 20% Compound 1 and 80% Soluplus;
scheme 1.4: 25% Compound 1 and 75% HPMCAS-HG;
scheme 1.5: 30% Compound 1 and 70% HPMCAS-HG.

4. A preparation method of the solid dispersion according to any one of claims 1 to 3, wherein the preparation method comprises the step of mixing the components;
preferably, the preparation method of the solid dispersion comprises the steps of: dissolving Compound 1 and the polymeric carrier in a solvent, mixing uniformly, and evaporating the solvent to obtain the solid dispersion; wherein the solvent is preferably evaporated by spray drying in the preparation method; the solvent used in the preparation method is preferably an organic solvent, more preferably selected from one or both of halogenated alkane solvents and alcohol solvents, and further preferably a mixture of dichloromethane and ethanol.

5. A solid dispersion prepared by the preparation method according to claim 4.

6. A pharmaceutical composition, wherein the pharmaceutical composition comprises the solid dispersion according to any one of claims 1 to 5, and one or more pharmaceutically acceptable carriers;
preferably, the pharmaceutical composition satisfies one or two of the following conditions:
(1) the excipient in the solid dispersion and the pharmaceutically acceptable carrier in the pharmaceutical composition are different from each other;
(2) the pharmaceutical composition is a tablet, a capsule, a granule, a powder, a dry suspension, or an enteric-coated preparation;
(3) the pharmaceutically acceptable carrier in the pharmaceutical composition is selected from one or more of a solubilizer, an acidic pH adjuster, a disintegrant, a glidant, a filler, a lubricant, and a surfactant.

7. The pharmaceutical composition according to claim 6, wherein the pharmaceutical composition comprises the following components in mass percentages:
20% to 70% of the solid dispersion according to any one of claims 1 to 5,
0% to 30% of a solubilizer,
0% to 20% of an acidic pH adjuster,
3% to 30% of a disintegrant,
0% to 10% of a glidant,
0% to 50% of a filler,
0% to 1% of a lubricant, and
0%-20% of a surfactant;
preferably, the pharmaceutical composition satisfies one or more of the following conditions:
(1) when the mass percentage of the solubilizer is 0%, the mass percentage of the acidic pH adjuster is not 0%;
(2) in the pharmaceutical composition, the mass percentage of the solid dispersion is 40%, 50%, 64.5%, or 66.4%; preferably 30% to 60%, more preferably 40% to 50%;
(3) in the pharmaceutical composition, the mass percentage of the solubilizer is 0% to 24%, such as 0%, 5.8%, 6%, 7.5%, 12%, 14%, 17.5%, or 24%; preferably 4% to 14%, such as 6%, 7.5%, or 12%;
(4) in the pharmaceutical composition, the mass percentage of the acidic pH adjuster is 0%, 5%, 6.25%, 10%, 15%, 20%, or 39.5%; preferably 0% to 15%, such as 0%, 5%, 6.25%, or 10%; more preferably 3% to 12%, such as 5%, 6.25%, or 10%;
(5) in the pharmaceutical composition, the mass percentage of the disintegrant is 3%, 8%, 10%, 14%, 26.4%, or 27.2%; preferably 3% to 20%, more preferably 3% to 15%, such as 3% or 10%;
(6) in the pharmaceutical composition, the mass percentage of the glidant is 0% or 4%; preferably 1% to 10%, such as 4%;
(7) in the pharmaceutical composition, the mass percentage of the filler is 0%, 13.5%, 16.5%, 17.5%, 18.50%, 19.25%, 19.5%, 21.5%, 22.5%, 25.5%, 26.5%, 28.5%, 29.25%, 29.5%, 30.5%, 34.5%, 35.5%, 36.5%, 39.5%, or 40.5%; preferably 10% to 45%, more preferably 20% to 40%, such as 28.5%, 29.25%, or 34.5%;
(8) in the pharmaceutical composition, the mass percentage of the lubricant is 0%, 0.4%, or 0.5%, preferably 0% or 0.5%;
(9) in the pharmaceutical composition, the mass percentage of the surfactant is 0% to 12%, preferably 0% to 10%, such as 0%, 1%, 5%, or 10%; more preferably 0% to 5%, such as 0%, 1%, or 5%;
(10) in the pharmaceutical composition, the mass ratio of the polymeric carrier to Compound 1 is 2:1 to 4:1, such as 2.3:1, 3:1, or 4:1; preferably 2:1 to 2.5:1, such as 2.3:1;
(11) in the pharmaceutical composition, the mass ratio of the acidic pH adjuster to Compound 1 is preferably 1:1 to 1:3, such as 1:1.2 or 1:2.4;
(12) in the pharmaceutical composition, the solubilizer is selected from one or more of PEG, PVP, HPMCAS, HPMC, Eudragit, and Soluplus, preferably selected from one or more of PVP, HPMC, and Soluplus, more preferably PVP or Soluplus; the PEG is preferably selected from one or both of PEG4000 and PEG6000; the PVP is preferably selected from one or more of PVP K17, PVP K30, PVP K90, and PVP-VA64, more preferably PVP-VA64; the HPMC is preferably HPMC E3LV; the HPMCAS is preferably selected from one or more of HPMCAS-LF, HPMCAS-MF, HPMCAS-HF, HPMCAS-LG, HPMCAS-MG, and HPMCAS-HG, more preferably HPMCAS-HG; the Eudragit is selected from one or both of Eudragit L100-55 and Eudragit S100;
(13) in the pharmaceutical composition, the acidic pH adjuster is selected from one or both of an organic acid and an inorganic acid; the organic acid is, for example, selected from one or more of citric acid, fumaric acid, malic acid, maleic acid, tartaric acid, succinic acid, oxalic acid, aspartic acid, mandelic acid, glutaric acid, and glutamic acid, preferably selected from one or more of fumaric acid, citric acid, tartaric acid, and malic acid, more preferably tartaric acid; the inorganic acid is preferably selected from one or more of hydrochloric acid, phosphoric acid, nitric acid, and sulfuric acid;
(14) in the pharmaceutical composition, the disintegrant is selected from one or more of CCMC-Na, CMS-Na, and CMC-Na, preferably one or both of CCMC-Na and CMS-Na, more preferably CMC-Na, or a mixture of CCMC-Na and CMS-Na;
(15) in the pharmaceutical composition, the glidant is colloidal silicon dioxide;
(16) in the pharmaceutical composition, the filler is selected from one or more of mannitol, lactose, compressible starch, and microcrystalline cellulose, preferably mannitol;
(17) in the pharmaceutical composition, the lubricant is magnesium stearate;
(18) in the pharmaceutical composition, the surfactant is selected from one or two of sodium dodecyl sulfate, vitamin E polyethylene glycol succinate, poloxamer 188, and poloxamer 407.

8. The pharmaceutical composition according to claim 7, wherein the pharmaceutical composition satisfies one or more of the following conditions:
(1) in the pharmaceutical composition, the PVP-VA64 is Plasdone^{™} S-630 or Plasdone^{™} S-630 ULTRA;
(2) in the pharmaceutical composition, when the disintegrant is a mixture of CCMC-Na and CMS-Na, the mass ratio of CCMC-Na to CMS-Na is 1:1 to 2:1, preferably 1.5:1;
(3) in the pharmaceutical composition, the solubilizer is selected from one or more of PVP-VA64, Soluplus, HPMC E3LV, and PVP K30, preferably PVP-VA64 or Soluplus;
(4) the acidic pH adjuster is selected from one or more of citric acid, fumaric acid, malic acid, and tartaric acid, preferably tartaric acid; when the acidic pH adjuster is tartaric acid, the mass percentage of the acidic pH adjuster is preferably 3% to 7%, more preferably 4% to 6%, such as 5%; when the acidic pH adjuster is malic acid, the mass percentage of the acidic pH adjuster is preferably 3% to 7%, more preferably 4% to 6%, such as 5%; when the acidic pH adjuster is fumaric acid, the mass percentage of the acidic pH adjuster is preferably 8% to 12%, more preferably 9% to 11%, such as 10%; when the acidic pH adjuster is citric acid, the mass percentage of the acidic pH adjuster is preferably 8% to 12%, more preferably 9% to 11%, such as 10%;
(5) the pharmaceutical composition consists of the solid dispersion, the solubilizer, the acidic pH adjuster, the disintegrant, the glidant, the filler, the lubricant, and the surfactant.

9. The pharmaceutical composition according to claim 6, wherein the pharmaceutical composition comprises components in mass percentages according to any one of the following schemes:
scheme 2.1: 19.35% Compound 1, 45.15% HPMCAS-HG, 5.8% PVP K90, 26.4% CMS-Na, 0.4% magnesium stearate, and 2.9% film coating premix (gastric-soluble);
scheme 2.2: 15% Compound 1, 35% HPMCAS-HG, 17.5% PVP-VA64, 4% colloidal silicon dioxide, 19.25% mannitol, 3% CMC-Na, and 6.25% tartaric acid;
scheme 2.3: 15% Compound 1, 35% HPMCAS-HG, 7.5% Soluplus, 4% colloidal silicon dioxide, 29.25% mannitol, 3% CMC-Na, and 6.25% tartaric acid;
scheme 2.4: 15% Compound 1, 35% HPMCAS-HG, 17.5% Soluplus, 4% colloidal silicon dioxide, 19.25% mannitol, 3% CMC-Na, and 6.25% tartaric acid;
scheme 2.5: 12% Compound 1, 28% HPMCAS-HG, 6% PVP-VA64, 4% colloidal silicon dioxide, 34.5% mannitol, 6% CCMC-Na, 4% CMS-Na, 5% tartaric acid, and 0.5% magnesium stearate;
scheme 2.6: 12% Compound 1, 28% HPMCAS-HG, 6% PVP-VA64, 4% colloidal silicon dioxide, 39.5% mannitol, 6% CCMC-Na, 4% CMS-Na, and 0.5% magnesium stearate;
scheme 2.7: 12% Compound 1, 28% HPMCAS-HG, 6% PVP-VA64, 4% colloidal silicon dioxide, 29.5% mannitol, 6% CCMC-Na, 4% CMS-Na, 10% tartaric acid, and 0.5% magnesium stearate;
scheme 2.8: 12% Compound 1, 28% HPMCAS-HG, 6% PVP-VA64, 4% colloidal silicon dioxide, 19.5% mannitol, 6% CCMC-Na, 4% CMS-Na, 20% tartaric acid, and 0.5% magnesium stearate;
scheme 2.9: 12% Compound 1, 28% HPMCAS-HG, 6% PVP-VA64, 4% colloidal silicon dioxide, 6% CCMC-Na, 4% CMS-Na, 39.5% tartaric acid, and 0.5% magnesium stearate;
scheme 2.10: 12% Compound 1, 28% HPMCAS-HG, 6% PVP-VA64, 4% colloidal silicon dioxide, 34.5% mannitol, 6% CCMC-Na, 4% CMS-Na, 5% fumaric acid, and 0.5% magnesium stearate;
scheme 2.11: 12% Compound 1, 28% HPMCAS-HG, 6% PVP-VA64, 4% colloidal silicon dioxide, 34.5% mannitol, 6% CCMC-Na, 4% CMS-Na, 5% citric acid, and 0.5% magnesium stearate;
scheme 2.12: 12% Compound 1, 28% HPMCAS-HG, 6% PVP-VA64, 4% colloidal silicon dioxide, 34.5% mannitol, 6% CCMC-Na, 4% CMS-Na, 5% malic acid, and 0.5% magnesium stearate;
scheme 2.13: 12% Compound 1, 28% HPMCAS-HG, 6% Soluplus, 4% colloidal silicon dioxide, 34.5% mannitol, 6% CCMC-Na, 4% CMS-Na, 5% tartaric acid, and 0.5% magnesium stearate;
scheme 2.14: 12% Compound 1, 28% HPMCAS-HG, 6% HPMC E3LV, 4% colloidal silicon dioxide, 34.5% mannitol, 6% CCMC-Na, 4% CMS-Na, 5% tartaric acid, and 0.5% magnesium stearate;
scheme 2.15: 12% Compound 1, 28% HPMCAS-HG, 6% PVP K30, 4% colloidal silicon dioxide, 34.5% mannitol, 6% CCMC-Na, 4% CMS-Na, 5% tartaric acid, and 0.5% magnesium stearate;
scheme 2.16: 12% Compound 1, 28% HPMCAS-HG, 4% colloidal silicon dioxide, 40.5% mannitol, 6% CCMC-Na, 4% CMS-Na, 5% citric acid, and 0.5% magnesium stearate;
scheme 2.17: 12% Compound 1, 28% HPMCAS-HG, 12% PVP-VA64, 4% colloidal silicon dioxide, 28.5% mannitol, 6% CCMC-Na, 4% CMS-Na, 5% citric acid, and 0.5% magnesium stearate;
scheme 2.18: 12% Compound 1, 28% HPMCAS-HG, 24% PVP-VA64, 4% colloidal silicon dioxide, 16.5% mannitol, 6% CCMC-Na, 4% CMS-Na, 5% citric acid, and 0.5% magnesium stearate;
scheme 2.19: 12% Compound 1, 28% HPMCAS-HG, 14% Soluplus, 4% colloidal silicon dioxide, 26.5% mannitol, 6% CCMC-Na, 4% CMS-Na, 5% tartaric acid, and 0.5% magnesium stearate;
scheme 2.20: 12% Compound 1, 28% HPMCAS-HG, 4% colloidal silicon dioxide, 35.5% mannitol, 6% CCMC-Na, 4% CMS-Na, 5% tartaric acid, 5% sodium lauryl sulfate, and 0.5% magnesium stearate;
scheme 2.21: 12% Compound 1, 28% HPMCAS-HG, 14% Soluplus, 25.5% mannitol, 6% CCMC-Na, 4% CMS-Na, 5% tartaric acid, 5% sodium lauryl sulfate, and 0.5% magnesium stearate;
scheme 2.22: 12% Compound 1, 28% HPMCAS-HG, 14% Soluplus, 4% colloidal silicon dioxide, 26.5% mannitol, 6% CCMC-Na, 4% CMS-Na, 5% sodium lauryl sulfate, and 0.5% magnesium stearate;
scheme 2.23: 19.92% Compound 1, 46.48% HPMCAS-HG, 6% PVP K30, 27.2% CMS-Na, and 0.4% magnesium stearate;
scheme 2.24: 12% Compound 1, 28% HPMCAS-HG, 14% Soluplus, 4% colloidal silicon dioxide, 26.5% mannitol, 10% CMS-Na, 5% tartaric acid, and 0.5% magnesium stearate;
scheme 2.25: 12% Compound 1, 28% HPMCAS-HG, 14% Soluplus, 4% colloidal silicon dioxide, 21.5% mannitol, 6% CCMC-Na, 4% CMS-Na, 5% tartaric acid, 5% sodium lauryl sulfate, and 0.5% magnesium stearate;
scheme 2.26: 12% Compound 1, 28% HPMCAS-HG, 14% PVP-VA64, 4% colloidal silicon dioxide, 21.5% mannitol, 6% CCMC-Na, 4% CMS-Na, 5% tartaric acid, 5% sodium lauryl sulfate, and 0.5% magnesium stearate;
scheme 2.27: 12% Compound 1, 28% HPMCAS-HG, 6% PVP-VA64, 4% colloidal silicon dioxide, 29.5% mannitol, 6% CCMC-Na, 4% CMS-Na, 5% tartaric acid, 5% sodium lauryl sulfate, and 0.5% magnesium stearate;
scheme 2.28: 12% Compound 1, 28% HPMCAS-HG, 6% PVP-VA64, 4% colloidal silicon dioxide, 36.5% mannitol, 6% CCMC-Na, 2% CMS-Na, 5% tartaric acid, and 0.5% magnesium stearate;
scheme 2.29: 12% Compound 1, 28% HPMCAS-HG, 6% PVP-VA64, 4% colloidal silicon dioxide, 30.5% mannitol, 6% CCMC-Na, 8% CMS-Na, 5% tartaric acid, and 0.5% magnesium stearate;
scheme 2.30: 15% Compound 1, 35% HPMCAS-HG, 4% colloidal silicon dioxide, 35.5% mannitol, 6% CCMC-Na, 4% CMS-Na, and 0.5% magnesium stearate;
scheme 2.31: 12% Compound 1, 28% HPMCAS-HG, 12% Soluplus, 4% colloidal silicon dioxide, 28.5% mannitol, 6% CCMC-Na, 4% CMS-Na, 5% tartaric acid, and 0.5% magnesium stearate;
scheme 2.32: 12% Compound 1, 28% HPMCAS-HG, 12% PVP-VA64, 4% colloidal silicon dioxide, 22.5% mannitol, 6% CCMC-Na, 4% CMS-Na, 10% tartaric acid, 0.5% magnesium stearate, and 1% poloxamer 407;
scheme 2.33: 12% Compound 1, 28% HPMCAS-HG, 12% PVP-VA64, 4% colloidal silicon dioxide, 18.5% mannitol, 6% CCMC-Na, 4% CMS-Na, 10% tartaric acid, 0.5% magnesium stearate, and 5% poloxamer 407;
scheme 2.34: 12% Compound 1, 28% HPMCAS-HG, 12% PVP-VA64, 4% colloidal silicon dioxide, 13.5% mannitol, 6% CCMC-Na, 4% CMS-Na, 10% tartaric acid, 0.5% magnesium stearate, and 10% poloxamer 407;
the pharmaceutical composition preferably comprises the components in mass percentages according to any one of schemes 2.2 to 2.34, and more preferably comprises the components in mass percentages according to any one of schemes 2.3, 2.5, and 2.31;
preferably, in schemes 2.1 to 2.34, Compound 1 and HPMCAS-HG are present as components in the form of a solid dispersion;
preferably, the pharmaceutical composition consists of the components in mass percentages according to any one of schemes 2.1 to 2.34, preferably the components in mass percentages according to any one of schemes 2.2 to 2.34, and more preferably the components in mass percentages according to any one of schemes 2.3, 2.5, and 2.31.

10. A preparation method of the pharmaceutical composition according to any one of claims 6 to 9, wherein the preparation method comprises the step of mixing the components;
preferably, the preparation method of the pharmaceutical composition comprises wet granulation or dry granulation;
more preferably, when the pharmaceutical composition is a tablet, the preparation method of the pharmaceutical composition comprises the steps of:
(1) subjecting the solid dispersion and the solubilizer to wet granulation, wet milling, drying, and dry milling to obtain a granule;
(2) uniformly mixing the granule with the remaining components, followed by tableting to obtain the tablet;
when the pharmaceutical composition is a tablet, the preparation method of the pharmaceutical composition comprises the steps of:
(1) subjecting the solid dispersion and the solubilizer to wet granulation, wet milling, drying, and dry milling to obtain a granule;
(2) uniformly mixing the granule with the remaining components, followed by tableting and coating to obtain the tablet;
when the pharmaceutical composition is a tablet, the preparation method of the pharmaceutical composition comprises the steps of:
(1) uniformly mixing one or more of the solid dispersion, the solubilizer, the glidant, the filler, the pH adjuster, the disintegrant, and the surfactant, followed by dry granulation and dry milling to obtain a granule;
(2) uniformly mixing the granule with the lubricant and the remaining components, followed by tableting to obtain the tablet;
when the pharmaceutical composition is a tablet, the preparation method of the pharmaceutical composition comprises the steps of:
(1) uniformly mixing one or more of the solid dispersion, the solubilizer, the glidant, the filler, the pH adjuster, the disintegrant, and the surfactant, followed by dry granulation and dry milling to obtain a granule;
(2) uniformly mixing the granule with the lubricant and the remaining components, followed by tableting and coating to obtain the tablet.

11. Use of the solid dispersion according to any one of claims 1 to 3 and 5 in the manufacture of a formulation of Compound 1; the formulation of Compound 1 is preferably the pharmaceutical composition according to any one of claims 6 to 9.

12. Use of one or more of the polymeric carrier according to any one of claims 1 to 3 and 5, the solubilizer according to any one of claims 6 to 9, the acidic pH adjuster according to any one of claims 6 to 9, the disintegrant according to any one of claims 6 to 9, and the surfactant according to any one of claims 6 to 9 for improving the solubility of Compound 1.

13. Use of the solid dispersion according to any one of claims 1 to 3 and 5 or the pharmaceutical composition according to any one of claims 6 to 9 in the manufacture of a medicament; the medicament is preferably for the treatment of one or more diseases selected from tumors, pulmonary fibrosis, autoimmune diseases, and neurodegenerative diseases; the medicament is more preferably for the treatment of one or more diseases selected from Alzheimer's disease, amyotrophic lateral sclerosis, multiple sclerosis, stroke, and neuromyelitis optica.
